# EUROPEAN PATENT SPECIFICATION

(11) **EP 4 229 214 B1**
(45) Date of publication and mention of the grant of the patent: **09.09.2026**
(21) Application number: 20957879.8
(22) Date of filing: 14.10.2020
(51) Int. Cl.: G16H 50/20, G16H 20/10, G16H 50/70, C12Q 1/68, G16B 20/00, G16B 25/00, G01N 33/575

(54) **METHODS AND SYSTEMS FOR PREDICTING IN-VIVO RESPONSE TO DRUG THERAPIES**
VERFAHREN UND SYSTEME ZUR VORHERSAGE DER IN-VIVO-REAKTION AUF ARZNEIMITTELTHERAPIEN
PROCÉDÉS ET SYSTÈMES POUR PRÉDIRE LA RÉPONSE IN-VIVO AUX THÉRAPIES MÉDICAMENTEUSES

(43) Date of publication of application: 23.08.2023
(73) Proprietor: ImpriMed, Inc., Palo Alto, CA 94303 (US)
(72) Inventor: BOHANNAN, Zachary Scott, Palo Alto, CA 94303 (US); LIM, Sungwon, Palo Alto, CA 94303 (US); PUDUPAKAM, Raghavendra Sumanth Kumar, Palo Alto, CA 94303 (US)
(74) Representative: Ostertag & Partner Patentanwälte mbB
(86) International application number: PCT/US2020/055599
(87) International publication number: WO 2022/081151

(56) References cited:
- WO-A1-2020/113237
- US-A1- 2008 118 576
- US-A1- 2009 177 450
- US-A1- 2010 106 475
- US-A1- 2010 280 987
- US-A1- 2011 014 644
- US-A1- 2011 275 089
- US-A1- 2015 038 340

## Description

### TECHNICAL FIELD

The disclosed implementations relate generally to providing predicted patient response to drug therapies and more specifically to systems and methods for predicting a patient's response to chemotherapy drug therapies.

### BACKGROUND

Determining a combination of drugs for drug therapies, such as a cocktail of anti-cancer drugs for chemotherapy that will be effective for a particular patient, can be a long process that is technically challenging. Currently, the efficacy of specific drugs is assessed based on disease progression in diseased sites before and after treatments. While this method provides a good indication of the *in-vivo* response and efficacy, it is a time consuming and financially costly approach that can take up to weeks, if not months, before returning results. Document WO2020113237 A1 discloses methods for training a machine learning model to predict effectiveness of a treatment for a disease or disorder of a subject having a particular set of biomarkers. This document does not disclose using ex vivo cell-viability data (initial and after drug exposure) nor longitudinal clinical response data (multiple responses over time) as input features for such prediction.

### SUMMARY

The invention is defined by the appended claims. Subject-matter which does not fall under the scope of the claims is not part of the invention.

The existing processes for determining or predicting expected patient response to drug therapies include tracking disease progression at diseased sites (e.g., tracking tumor size) before and after treatment, which may be financially costly and time consuming. Personalized predictive modeling is a promising approach to overcome the limitations of conventional drug efficacy testing methods. This methodology decreases the time to efficacy prediction from weeks or months to the order of days. This allows patients to wait for predicted drug efficacy results and for physicians to prescribe drugs to a patient based on the patient's personalized predicted drug efficacy results, thereby improving the patient's chances of responding positively to the drug therapy without significant delay to starting the drug therapy.

In general, predictive models require a large amount of data in order to train the predictive models to provide robust results. Such a large amount of data may be hard to acquire due to the number of people (or animals, such as dogs) undergoing such drug therapies. Additionally, most predictive models are trained to provide results for single agent therapies (e.g., drug therapies that include only one drug) and thus fail to consider the effect of a combination of drugs (e.g., such as in multiple agent drug therapies). Therefore, they fail to provide a robust prediction of a patient's *in-vivo* response to drug therapies that include a combination of two or more drugs.

Accordingly, there is a need for tools that can accurately calculate and predict a patient's *in-vivo* response to different drug therapies (e.g., a likelihood that a patient will have a positive response to drug therapies), including single agent drug therapies and multiple agent drug therapies. There is also a need for tools that employ such calculations and predictions to provide personalized prescription of drug therapies to patients.

One solution is to train predictive models to provide predicted patient response to different drug therapies (including single agent drug therapies and multiple agent drug therapies). For each patient, functional, genetic, and clinical data is used to provide predicted *in-vivo* response in a cost effective and timely manner. This technique produces (e.g., generates or provides) predictions based on predicted *in-vivo* response to different drug therapies based on the functional, genetic, and clinical data of each patient, thereby providing robust predictions that can guide drug therapy prescription for improved patient response and improved drug therapy efficacy.

With medical treatments, especially in chemotherapies that involve one or more anti-cancer drugs, time can often be of the essence and starting a patient on a drug therapy treatment course as soon as possible can make a difference in the treatment outcome and disease prognosis. Additionally, identifying an effective drug or combination of drugs to which the patient has a positive response can be challenging and time consuming. Thus, the ability to provide fast and robust predictions of patients' *in-vivo* response to drug therapies can lead to lives saved, faster recovery, and improved quality of care.

In accordance with some implementations, a method for building models for predicting patient response to drug therapies executes at an electronic device with a display, one or more processors, and memory. For example, the electronic device can be a smart phone, a tablet, a notebook computer, a desktop computer, an individual server computer, or a server system (e.g., running in the cloud). For each patient of a first plurality of patients, the device retrieves respective functional data and respective clinical data corresponding to the respective patient. The respective functional data includes initial cell viability and cell viability in response to exposure to one or more drug therapies, and the respective clinical data includes patient information over time. For each of the patients, the device forms a respective feature vector that includes the respective functional data and the respective clinical data corresponding to the respective patient. The device then uses at least a first subset of the feature vectors to train a first model to predict individual patient response to a first drug therapy. The device then stores the trained first model in a database for subsequent use in predicting patient response to the first drug therapy.

In some implementations, for each patient of the first plurality of patients, the device retrieves respective genetic data corresponding to the respective patient. The respective genetic data includes information obtained from deoxyribonucleic acid (DNA) and ribonucleic acid (RNA) extracted from cells obtained from a diseased site of the respective patient. The respective feature vector further includes the respective genetic data corresponding to the respective patient.

In some implementations, the respective genetic data also includes information obtained from a DNA sequence extracted from non-cancerous cells obtained from a healthy site of the respective patient and information obtained from an RNA sequence extracted from non-cancerous cells obtained from a healthy site of the respective patient.

In some implementations, the respective genetic data includes information regarding: RNA transcripts, DNA variants, genes, and pathways.

In some implementations, the respective genetic data includes information measuring one or more of: the presence of genetic mutations, variant allele frequency, and a number of variant alleles.

In some implementations, the respective genetic data includes information regarding at least 100 genes.

In some implementations, the respective functional data includes information obtained from live cells extracted from a tumor site of the respective patient, and the respective functional data includes one or more of: physical integrity of the live cells, metabolic activity of the live cells, mechanical activity of the live cells, mitotic activity of the live cells, and proliferation capacity of the live cells for a predetermined cellular phenotype.

In some implementations, the respective functional data includes information obtained from live cells extracted from a tumor site of the respective patient, and the respective functional data includes one or more of a size distribution of the live cells, a shape distribution of the live cells, a distribution of the live cells with respect to expression of a biomarker, and phenotypic features of the live cells.

In some implementations, the respective functional data includes information obtained from live cells extracted from a tumor site of the respective patient, the first drug therapy includes at least a first drug, and the respective functional data includes one or more of: a measure of the potency of one or more first drugs for inhibiting a predetermined biochemical function, a maximum cytotoxicity of the one or more first drugs, an area under a curve determined using a plot of cell viability in response to dosage of the one or more first drugs, and the one or more first drugs includes at least the first drug

In some implementations, for each patient of a second plurality of patients, the device retrieves respective functional data and respective clinical data corresponding to the respective patient of the second plurality of patients. The respective functional data corresponding to the respective patient of the second plurality of patients includes initial cell viability and cell viability in response to exposure to one or more drug therapies. The respective functional data corresponding to the respective patient of the second plurality of patients data includes one or more of: a measure of the potency of one or more second drugs for inhibiting a predetermined biochemical function, a maximum cytotoxicity of the one or more second drugs, and an area under a curve determined using a plot of cell viability in response to dosage of the one or more second drugs. The one or more second drugs differs from the one or more first drugs by at least one drug, the one or more second drugs includes a second drug that is different from the first drug, and the respective clinical data corresponding to the respective patient of the second plurality of patients includes patient information over time. The device forms a respective feature vector that includes the respective functional data and respective clinical data corresponding to the respective patient of the second plurality of patients. The device uses at least a second subset of the feature vectors corresponding to the respective patient of the second plurality of patients to train a second model to predict individual patient response to a second drug therapy that is different from the first drug therapy. The device then stores the trained second model in a database for subsequent use in predicting patient response to the second drug therapy. The second drug therapy is distinct from the first drug therapy and includes at least the second drug.

In some implementations, the device stores the trained first model and the trained second model in a database for subsequent use in predicting patient response to a third drug therapy that includes at least the first drug of the first drug therapy and the second drug of the second drug therapy.

In some implementations, the respective clinical data includes one or more of: an age of the respective patient, a sex of the respective patient, a weight of the respective patient, a diagnosis date, patient information over time, an indicator regarding whether or not the patient has relapsed, an indicator of the respective patient's response to a second drug therapy, a stage of the respective patient's disease progression, a concentration of total protein, a concentration of one or more biochemicals, an indicator of the drug therapy the respective patient is receiving, a tumor size, and an indication of other health conditions associated with the respective patient.

In some implementations, the one or more drug therapies are one or more chemotherapies, and each chemotherapy includes one or more drugs for treating cancer.

In some implementations, the device determines that each of the respective functional data and respective clinical data is complete, and in accordance with a determination that at least one of the respective functional data and respective clinical data includes one or more missing values, the device replaces at least one of the one or more missing values with an inferred value.

In some implementations, the feature vectors are used to train the first model to output a prediction interval of the predicted individual patient response to the first drug therapy.

In some implementations, the first drug therapy includes a predefined combination of two or more drugs.

In some implementations, the first subset of the feature vectors is a subset, less than all, of the feature vectors. The device uses a second subset of the feature vectors, distinct from the first subset of the feature vectors, to test the trained model.

In some implementations, at least a first subset of the plurality of patients includes patients that have undergone one or more drug therapies that includes the first drug therapy.

In some implementations, the one or more drug therapies associated with the first subset of the plurality of patients includes one or more drug therapies that are different from the first drug therapy.

In some implementations, the plurality of patients further include a second subset of patients that have undergone one or more drug therapies that includes drugs other than the first drug.

In some implementations, the plurality of patients further includes a second subset of patients that have undergone one or more drug therapies that are different from the one or more drug therapies associated with the first subset of patients, and the one or more drug therapies associated with the second subset of patients do not include the first drug therapy.

In accordance with some implementations, a method of predicting patient response to one or more drug therapies executes at an electronic device with a display, one or more processors, and memory. For example, the electronic device can be a smart phone, a tablet, a notebook computer, a desktop computer, a server computer, a system of server computers, or a wearable device such as a smart watch. The device identifies a patient having a first disease condition, and retrieves a first trained model built to predict patient response to a first drug therapy for treating the first disease condition. The first trained model has been trained according to data for a plurality of previous patients. Each previous patient provided medical data during drug therapy that includes one or more drugs, and at least a first subset of the previous patients underwent one or more drug therapies that includes the first drug therapy. The device then receives medical data for the patient. The medical data includes functional data and clinical data corresponding to features used by the first trained model. The functional data includes initial cell viability, and the clinical data includes patient information over time. The device extracts, from the medical data, features corresponding to the features used by the first trained model. The device then forms a feature vector comprising the extracted features, applies the first trained model to the feature vector to generate a prediction of the patient's response to the first drug therapy, and provides the predicted patient's response to the first drug therapy.

In some implementations, the medical data further includes genetic data, including information obtained from a DNA sequence extracted from a tumor of the patient, and the feature vector includes one or more features computed according to the genetic data.

In some implementations, the first trained model also generates a prediction interval of the predicted patient's response to the first drug therapy, and the device provides the prediction interval of the predicted patient's response to the first drug therapy.

In some implementations, the prediction of the patient's response to the first drug therapy includes a probability (e.g., likelihood) of a positive response to the first drug therapy.

In some implementations, the device applies a second trained model to the feature vector to generate a prediction of the patient's response to a second drug therapy, and the device provides the predicted patient's response to the second drug therapy. The second trained model is different from the first trained model, and the second drug therapy includes at least one drug that is different from one or more drugs in the first drug therapy.

In some implementations, the prediction of the patient's response to the second drug therapy includes a probability (e.g., likelihood) of a positive response to the second drug therapy.

In some implementations, the first drug therapy includes a predefined combination of two or more drugs.

In some implementations, the first model includes a plurality of decision trees, and the device forms an aggregate prediction for the first drug therapy using a random forest of the plurality of decision trees.

In some implementations, the one or more drug therapies associated with the first subset of the previous patients includes one or more drug therapies that are different from the first drug therapy.

In some implementations, the previous patients further include a second subset that underwent one or more drug therapies that includes drugs other than the first drug.

In some implementations, the previous patients further include a second subset that underwent one or more drug therapies that are different from the one or more drug therapies associated with the first subset, and the one or more drug therapies associated with the second subset do not include the first drug therapy.

Typically, an electronic device includes one or more processors, memory, a display, and one or more programs stored in the memory. The programs are configured for execution by the one or more processors and are configured to perform any of the methods described herein.

In some implementations, a non-transitory computer readable storage medium stores one or more programs configured for execution by a computing device having one or more processors, memory, and a display. The one or more programs are configured to perform any of the methods described herein.

Thus methods and systems are disclosed for building (e.g., training) models for predicting patient response to drug therapies and for using the trained models for predicting patient response to one or more drug therapies.

Both the foregoing general description and the following detailed description are exemplary and explanatory, and are intended to provide further explanation of the technology.

### BRIEF DESCRIPTION OF THE DRAWINGS

For a better understanding of these systems, methods, and graphical user interfaces, as well as additional systems, methods, and graphical user interfaces that correlate patients with treating clinicians, refer to the Description of Implementations below, in conjunction with the following drawings, in which like reference numerals refer to corresponding parts throughout the figures.
Figure 1A illustrates training one or more predictive models in accordance with some implementations.
Figure 1B illustrates using one or more predictive models in accordance with some implementations.
Figure 2A is a block diagram illustrating a computing device according to some implementations.
Figure 2B is a block diagram illustrating a server according to some implementations.
Figures 3A - 3B illustrate how a predictive model is trained according to some implementations.
Figures 4A - 4D provide examples of functional data according to some implementations.
Figure 5 provides examples of clinical data according to some implementations.
Figure 6A provides an example of variants in cancerous cells and non-cancerous cells according to some implementations.
Figures 6B - 6D provide examples of genetic data according to some implementations.
Figures 6E - 6G provide examples of how pathways, genes, and variants can correspond to one another in accordance with some implementations.
Figure 7A illustrates using one or more predictive models for predicting patient response to one or more drug therapies according to some implementations.
Figure 7B illustrates predicted patient responses to drug therapies according to some implementations.
Figures 8A - 8G provide a flow diagram of a method for building a predictive model for predicting patient response to drug therapies according to some implementations.
Figures 9A - 9C provide a flow diagram of a method for predicting patient response to drug therapies according to some implementations.

Reference will now be made to implementations, examples of which are illustrated in the accompanying drawings. In the following description, numerous specific details are set forth in order to provide a thorough understanding of the present technology. However, it will be apparent to one of ordinary skill in the art that the present technology may be practiced without requiring these specific details.

### DESCRIPTION OF IMPLEMENTATIONS

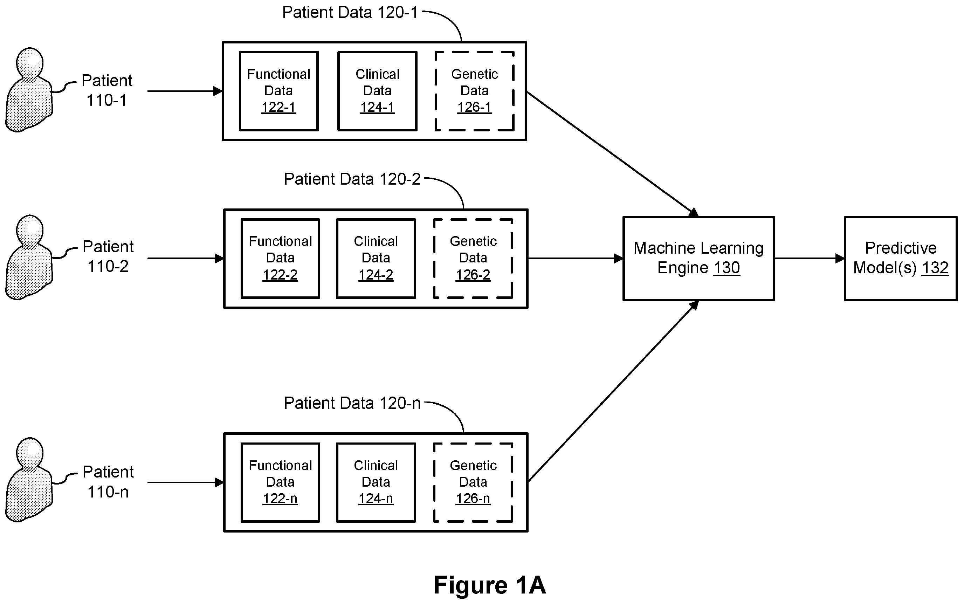

Figure 1A illustrates training one or more predictive model(s) 132 using patient data 120 from a plurality of patients 110 (e.g., previous patients, patients who have previously undergone one or more drug therapies, or patients who are currently being treated with one or more drug therapies). The patient data 120 is input into a machine learning engine 130 configured to train (e.g., produce, generate) one or more predictive models 132 for predicting a patient's response to one or more drug therapies.

The plurality of patients includes patients 110 of a same species. For example, the plurality of patients may include patients 110 that are all dogs (of any breed). In another example, the plurality of patients may include patients 110 that are all humans. The species of the patients in the plurality of patients determines the species for which the one or more predictive model(s) 132 are trained to provide a prediction. For example, when the plurality of patients include patients that are cats, the one or more predictive model(s) 132 trained using the patient data 120 corresponding to the plurality of patients 110 (e.g., plurality of cats) are trained to provide predicted response(s) of a specific cat to one or more drug therapies.

The patient data 120 (e.g., medical data or medical information) is obtained for each patient in the plurality of patients. The patient data 120 includes functional data 122 and clinical data 124. In some implementations, the patient data 120 also includes genetic data 126. For example, the first patient data 120-1 (e.g., medical data or medical information) is obtained for the first patient 110-1. The patient data 120-1 includes functional data 122-1, clinical data 124-1, and optionally, genetic data 126-1 corresponding to the first patient 110-1.

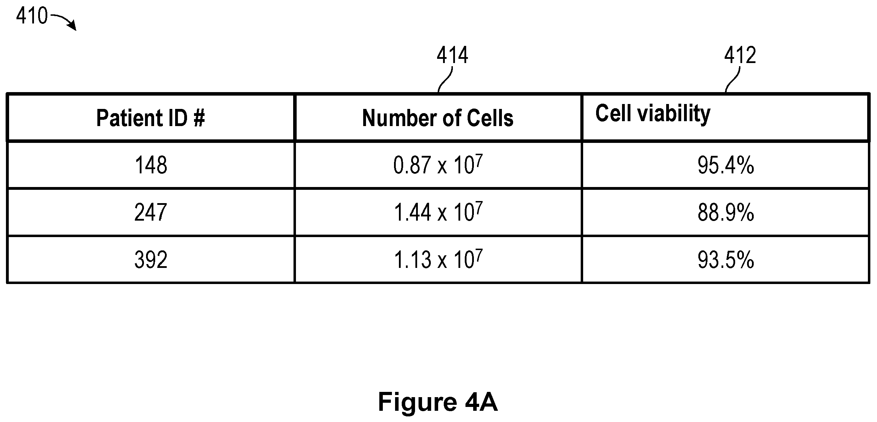

The functional data 122 includes cell viability information and cell drug sensitivity information that is obtained using a live cell sample biopsied from a diseased site (e.g., tumor site) of the patient. The clinical data 124 includes medical and demographic information regarding the patient. For example, the clinical data may include information such as age, gender, total protein concentration in blood, concentration of one or more biomarkers in blood, etc. The genetic data 126 includes information regarding deoxyribonucleic acid (DNA) and ribonucleic acid (RNA) extracted from live cells obtained (e.g., via biopsy) from a diseased site (e.g., tumor site) of the patient. Additional details regarding the functional data 122, the clinical data 124, and the genetic data 126 are provided with respect to Figures 4A - 4D, 5, and 6A - 6B, respectively.

The machine learning engine 130 forms a feature vector for each respective patient of the plurality of patients using the functional data 122, the clinical data 124, and optionally, the genetic data 126 corresponding to the respective patient. The machine learning engine 130 then uses the feature vectors to train the one or more predictive models 132 so that the predictive models 132 can predict a patient's response to one or more drug therapies.

In some implementations, the one or more predictive models 132 includes a plurality of predictive models (e.g., a first predictive model and a second predictive model) and each model of the one or more predictive models 132 is trained to provide a predicted patient's response to a specific drug therapy. For example, a first predictive model may be trained to provide a predicted patient's response to a first drug therapy and a second predictive model may be trained to provide a predicted patient's response to a second drug therapy that is different from the first drug therapy. For instance, the first drug therapy may include a first drug and the second drug therapy may include a predetermined combination of drugs or may include a second drug that is different from the first drug. In some implementations, the predetermined combination of drugs may include drugs other than the first drugs. In some implementations, the predetermined combination of drugs includes the first drug as well as one or more other drugs. In some implementations, the predetermined combination of drugs includes the drugs other than the first drugs and the predetermined combination of drugs does not include the first drug.

In some implementations, the first model is trained using a first plurality of patients and the second model is trained using a second plurality of patients that is different from the first plurality of patients. For example, the first plurality of patients includes patients who have been or are currently treated with one or more drug therapies and the one or more drug therapies associated with the first plurality of patients includes the first drug therapy. In contrast, the second plurality of patients includes patients who have been or are currently treated with one or more drug therapies and the one or more drug therapies associated with the second plurality of patients includes the second drug therapy. The first plurality of patients differs from the second plurality of patients by at least one patient. In some implementations, the first plurality of patients includes one or more patients in common with the second plurality of patients.

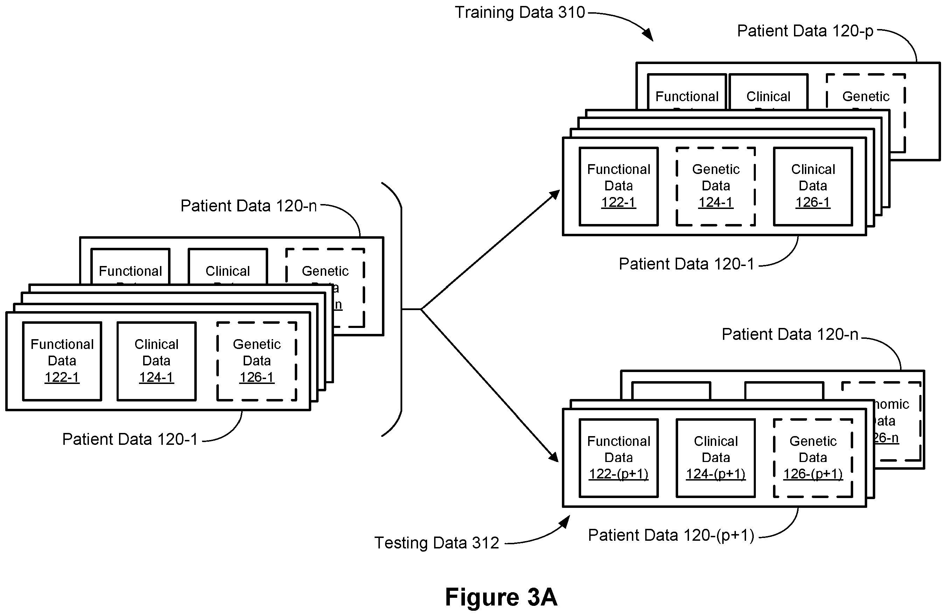

Additional details regarding training the one or more predictive models 132 is provided with respect to Figure 3A.

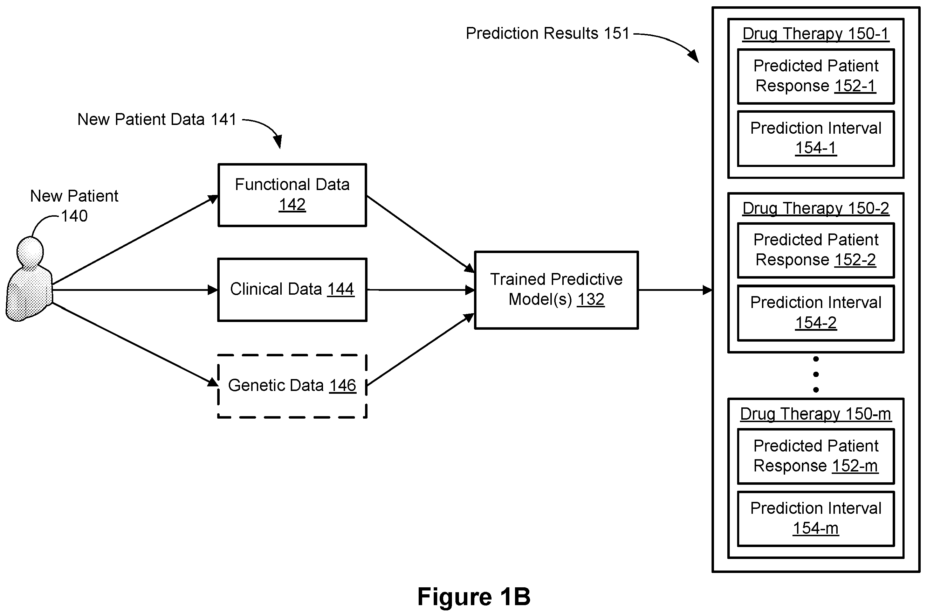

Figure 1B illustrates using one or more trained predictive models 132 that are trained to predict a patient's response to one or more drug therapies. When a new patient 140 needs their potential response to drug therapy options assessed, the new patient 140 provides the predictive model(s) 132 with new patient data 141 corresponding to the new patient. The new patient data 141 includes functional data 142 and clinical data 144 corresponding to the new patient 140. In some implementations, the new patient data 141 also includes genetic data 146 corresponding to the new patient 140. The new patient data 141 is provided as input to the one or more trained predictive models 132, and the one or more trained predictive models 132 output prediction results 151 for one or more different drug therapies. The one or more trained predictive models 132 outputs a plurality of predicted patient responses 152. In some implementations, the one or more trained predictive models 132 also output a corresponding prediction interval 154 for a given drug therapy 150. In some implementations, the one or more trained predictive models 132 output an accuracy score, confidence value, or p-value of the predicted patient response 152. For example, as shown in Figure 1B, the plurality of predicted patient responses 152 includes a first predicted patient response 152-1 and a corresponding prediction interval 154-1 for a first drug therapy 150-1, and a second predicted patient response 152-2 and a corresponding prediction interval 154-2 for a second drug therapy 150-2 that is different from the first drug therapy 150-1. Additional details regarding the functional data 142, the clinical data 144, and the genetic data 146 of the new patient data 141 are provided with respect to Figures 4A - 4D, 5, and 6A - 6B, respectively.

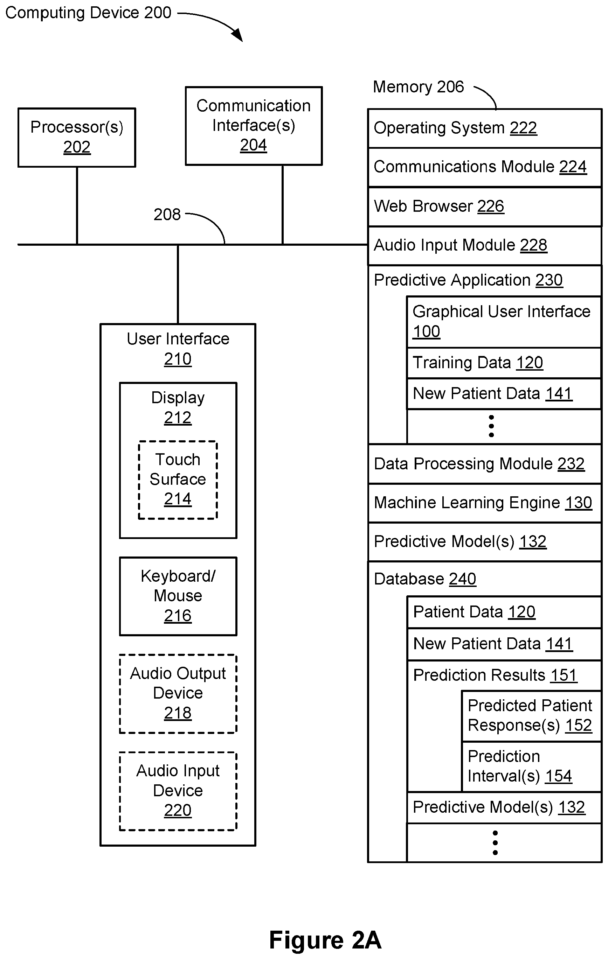

Figure 2A is a block diagram illustrating a computing device 200, corresponding to a computing system, which can train and/or execute predictive model(s) 132 in accordance with some implementations. Various examples of the computing device 200 include a desktop computer, a laptop computer, a tablet computer, a server computer, a server system, a wearable device such as a smart watch, and other computing devices that have a processor capable of training and/or running predictive model(s) 132. The computing device 200 may be a data server that hosts one or more databases, models, or modules, or may provide various executable applications or modules. The computing device 200 typically includes one or more processing units (processors or cores) 202, one or more network or other communications interfaces 204, memory 206, and one or more communication buses 208 for interconnecting these components. The communication buses 208 optionally include circuitry (sometimes called a chipset) that interconnects and controls communications between system components. The computing device 200 typically includes a user interface 210. The user interface 210 typically includes a display device 212 (e.g., a screen or monitor). In some implementations, the computing device 200 includes input devices such as a keyboard, mouse, and/or other input buttons 216. Alternatively or in addition, in some implementations, the display device 212 includes a touch-sensitive surface 214, in which case the display device 212 is a touch-sensitive display. In some implementations, the touch-sensitive surface 214 is configured to detect various swipe gestures (e.g., continuous gestures in vertical and/or horizontal directions) and/or other gestures (e.g., single/double tap). In computing devices that have a touch-sensitive display 214, a physical keyboard is optional (e.g., a soft keyboard may be displayed when keyboard entry is needed). The user interface 210 also includes an audio output device 218, such as speakers or an audio output connection connected to speakers, earphones, or headphones. Furthermore, some computing devices 200 use a microphone 220 and voice recognition software to supplement or replace the keyboard. An audio input device 220 (e.g., a microphone) captures audio (e.g., speech from a user).

The memory 206 includes high-speed random-access memory, such as DRAM, SRAM, DDR RAM, or other random-access solid-state memory devices; and may include non-volatile memory, such as one or more magnetic disk storage devices, optical disk storage devices, flash memory devices, or other non-volatile solid-state storage devices. In some implementations, the memory 206 includes one or more storage devices remotely located from the processors 202. The memory 206, or alternatively the non-volatile memory devices within the memory 206, includes a non-transitory computer-readable storage medium. In some implementations, the memory 206 or the computer-readable storage medium of the memory 206 stores the following programs, modules, and data structures, or a subset or superset thereof:
- an operating system 222, which includes procedures for handling various basic system services and for performing hardware dependent tasks;
- a communications module 224, which is used for connecting the computing device 200 to other computers and devices via the one or more communication network interfaces 204 (wired or wireless), such as the Internet, other wide area networks, local area networks, metropolitan area networks, and so on;
- a web browser 226 (or other application capable of displaying web pages), which enables a user to communicate over a network with remote computers or devices;
- an audio input module 228 (e.g., a microphone module) for processing audio captured by the audio input device 220. The captured audio may be sent to a remote server and/or processed by an application executing on the computing device 200 (e.g., predictive application 230);
- a predictive application 230, which includes a graphical user interface 100 that allows a user to navigate the predictive application 230, such as accessing and editing patient data 120, including functional data 122, clinical data 124, and genetic data 126, and/or accessing and editing new patient data 141, including functional data 142, clinical data 144, and genetic data 146. For example, a new patient 140 or the new patient's physician may use the graphical user interface 100 of the predictive application 230 to provide patient data 141, such as demographic information (age, gender, etc.) in the clinical data 144 or to upload medical charts that include patient clinical data 144. In another example, one or more users may use the graphical user interface 100 of the predictive application 230 to replace missing data values in the patient data 120 with imputed (e.g., inferred) values. The patient data 120 is then compiled by the machine learning engine 130 in order to train predictive model(s) 132. The predictive application 230 may also input new patient data 141 into the predictive model(s) 132 and utilize the predictive model(s) 132 to predict the patient's response to drug therapies. The predictive model(s) 132 take patient data (including functional data 142, clinical data 144, and genetic data 146) into account when generating the prediction results 151 of predicted patient response 152 to different drug therapies 150. In some implementations, the prediction results 151 of predicted patient response 152 to different drug therapies 150 includes predicted patient response 152 for single agent therapies (e.g., a single drug) as well as multi-agent therapies (e.g., a predefined combination of two or more drugs);
- a data processing module 232 configured to perform preprocessing steps necessary to convert any raw information into correct data types for the patient data 120 or for the new patient data 141. For example, the data processing module 232 may be configured to perform optical character recognition (OCR) or natural language processing to convert and extract clinical data 124 or 144 from a patient's medical chart or medical history documents. The data processing module 232 may also be configured to perform one or more calculations based on the received raw data in order to generate a data value for the patient data 120 or the new patient data 141. For example, the data processing module 232 may perform one or more calculations to determine a total patient response based on multiple reported patient responses over time. The data processing module 232 may also be configured to generate imputed (e.g., inferred) data to replace missing values in the patient data 120 or the new patient data 141. The data processing module 232 may utilize a variety of different methods to generate (e.g., determine or calculate) the imputed data. In some implementations, the imputation or inference method used by the data processing module 232 to generate the imputed data is based at least in part on the type of data that is missing;
- a machine learning engine 130 configured to train the predictive model(s) 132 using the patient data 120 (including functional data 122, clinical data 124, and genetic data 126) as inputs for training the predictive model(s) 132;
- one or more predictive models 132 trained by machine learning engine 130 to provide prediction results 151 including predicted patient's response(s) 152 to different drug therapies and prediction interval(s) 154 (e.g., a confidence interval or a p-value);
- a database 240, which stores information, such as patient data 120, new patient data 141, prediction results 151 (including predicted patient response(s) 152 and prediction interval(s) 154), and one or more predictive model(s) 132. Patient data 120 includes functional data 122, clinical data 124, and genetic data 126, details of which are provided with respect to Figures 4A - 4D, 5, and 6A - 6B, respectively. New patient data 141 includes functional data 142, clinical data 144, and genetic data 146, details of which are provided with respect to Figures 4A - 4D, 5, and 6A - 6B, respectively. In some implementations, the patient information includes social determinants, such as homelessness.

In some implementations, the memory 206 stores metrics and/or scores determined by the one or more predictive models 132. In addition, the memory 206 may store thresholds and other criteria, which are compared against the metrics and/or scores determined by the machine learning engine 130 and/or predictive model(s) 132. For example, the predictive model(s) 132 may determine (e.g., calculate) a prediction interval 154 (e.g., a confidence value, an accuracy score or a p-value) for each predicted patient response 152.

Each of the above identified executable modules, applications, or sets of procedures may be stored in one or more of the previously mentioned memory devices, and corresponds to a set of instructions for performing a function described above. The above identified modules or programs (i.e., sets of instructions) need not be implemented as separate software programs, procedures, or modules, and thus various subsets of these modules may be combined or otherwise re-arranged in various implementations. In some implementations, the memory 206 stores a subset of the modules and data structures identified above. Furthermore, the memory 206 may store additional modules or data structures not described above.

Although Figure 2A shows a computing device 200, Figure 2A is intended more as a functional description of the various features that may be present rather than as a structural schematic of the implementations described herein. In practice, and as recognized by those of ordinary skill in the art, items shown separately could be combined and some items could be separated.

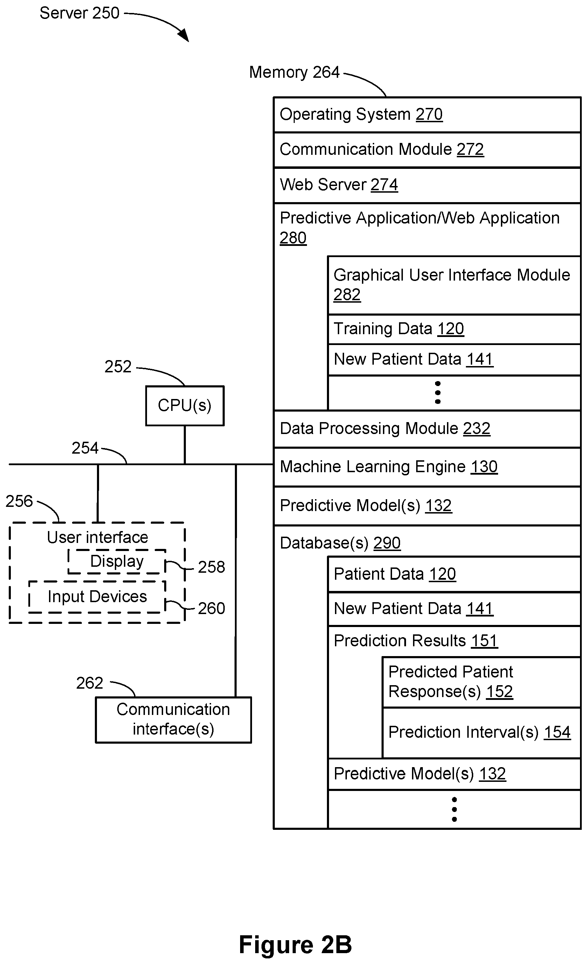

Figure 2B is a block diagram of a server 250 in accordance with some implementations. A server 250 may host one or more databases 290 or may provide various executable applications or modules. A server 250 typically includes one or more processing units/cores (CPUs) 252, one or more network interfaces 262, memory 264, and one or more communication buses 254 for interconnecting these components. In some implementations, the server 250 includes a user interface 256, which includes a display 258 and one or more input devices 260, such as a keyboard and a mouse. In some implementations, the communication buses 254 include circuitry (sometimes called a chipset) that interconnects and controls communications between system components.

In some implementations, the memory 264 includes high-speed random-access memory, such as DRAM, SRAM, DDR RAM, or other random-access solid-state memory devices, and may include non-volatile memory, such as one or more magnetic disk storage devices, optical disk storage devices, flash memory devices, or other non-volatile solid-state storage devices. In some implementations, the memory 264 includes one or more storage devices remotely located from the CPU(s) 252. The memory 264, or alternatively the non-volatile memory devices within the memory 264, comprises a non-transitory computer readable storage medium.

In some implementations, the memory 264, or the computer readable storage medium of the memory 264, stores the following programs, modules, and data structures, or a subset thereof:
- an operating system 270, which includes procedures for handling various basic system services and for performing hardware dependent tasks;
- a network communication module 272, which is used for connecting the server 250 to other computers via the one or more communication network interfaces (wired or wireless) and one or more communication networks, such as the Internet, other wide area networks, local area networks, metropolitan area networks, and so on;
- a web server 274 (such as an HTTP server), which receives web requests from users and responds by providing responsive web pages or other resources;
- a predictive application or a predictive web application 280, which may be downloaded and executed by a web browser 226 on a user's computing device 200. In general, a predictive web application 280 has the same functionality as a desktop predictive application 230, but provides the flexibility of access from any device at any location with network connectivity, and does not require installation and maintenance. In some implementations, the predictive web application 280 includes various software modules to perform certain tasks. In some implementations, the predictive web application 280 includes a graphical user interface module 282, which provides the user interface for all aspects of the predictive web application 280. In some implementations, the predictive web application 280 includes patient data 120 and new patient data 141 as described above for a computing device 200;
- a data processing module 232 for performing preprocessing steps required to convert raw information into correct data types for the patient data 120 or for the new patient data 141, performing one or more calculations based on the received raw data in order to generate a data value for the patient data 120 or the new patient data 141, and/or generate imputed (e.g., inferred) data to replace missing values in the patient data 120 or the new patient data 141 as described above;
- a machine learning engine 130 for training the predictive model(s) 132 as described above;
- one or more predictive models 132 trained to provide prediction results 151 as described above;
- one or more databases 290, which store data used or created by the predictive web application 280 or predictive application 230. The databases 290 may store patient data 120, new patient data 141, prediction results 151 (including predicted patient response(s) 152 to drug therapies and corresponding prediction interval(s) 154), and one or more predictive module(s) 132 as described above.

Each of the above identified executable modules, applications, or sets of procedures may be stored in one or more of the previously mentioned memory devices, and corresponds to a set of instructions for performing a function described above. The above identified modules or programs (i.e., sets of instructions) need not be implemented as separate software programs, procedures, or modules, and thus various subsets of these modules may be combined or otherwise re-arranged in various implementations. In some implementations, the memory 264 stores a subset of the modules and data structures identified above. In some implementations, the memory 264 stores additional modules or data structures not described above.

Although Figure 2B shows a server 250, Figure 2B is intended more as a functional description of the various features that may be present rather than as a structural schematic of the implementations described herein. In practice, and as recognized by those of ordinary skill in the art, items shown separately could be combined and some items could be separated. In addition, some of the programs, functions, procedures, or data shown above with respect to a server 250 may be stored or executed on a computing device 200. In some implementations, the functionality and/or data may be allocated between a computing device 200 and one or more servers 250. Furthermore, one of skill in the art recognizes that Figure 2B need not represent a single physical device. In some implementations, the server functionality is allocated across multiple physical devices that comprise a server system. As used herein, references to a "server" include various groups, collections, or arrays of servers that provide the described functionality, and the physical servers need not be physically collocated (e.g., the individual physical devices could be spread throughout the United States or throughout the world).

Figures 3A - 3B illustrate how a predictive model (e.g., a first predictive model) of the plurality of predictive models 132 are trained according to some implementations. In order to train the predictive model of the plurality of predictive models 132, the machine learning engine 130 receives patient data 120 (e.g., training data) for a plurality of patients (e.g., n number of patients, patients 110-1 to 110-n. The patient data 120 for a respective patient of the plurality of patients 110 includes functional data 122, clinical data 124, and optionally, genetic data 126. The machine learning engine 130 divides the patient data 120 into a first subset of patient data 120 to be used as training data 310 and a second subset of patient data 120 to be used as testing data 312. For example, as shown in Figure 3A, the first subset of patient data (e.g., the training data 310, patient data 120-1 to 120-p, where p < n) includes information corresponding to a first subset of patients (e.g., patients 110-1 to 110-p) and the second subset of patient data (e.g., the testing data 312, patient data 120-(p+1) to 120-n) includes information corresponding to a second subset of patients (e.g., patients 110-(p+1) to 110-n). In some implementations, the training data 310 (e.g., the first subset of patient data) includes at least 50%, 60%, 70%, 80%, or 90% of the plurality of patient data 120. For example, the training data 310 (e.g., the first subset of the plurality of patient data) may include 70% of the plurality of patient data 120 and the testing data 312 (e.g., the second subset of the plurality of patient data) includes 30% of the plurality of patient data 120.

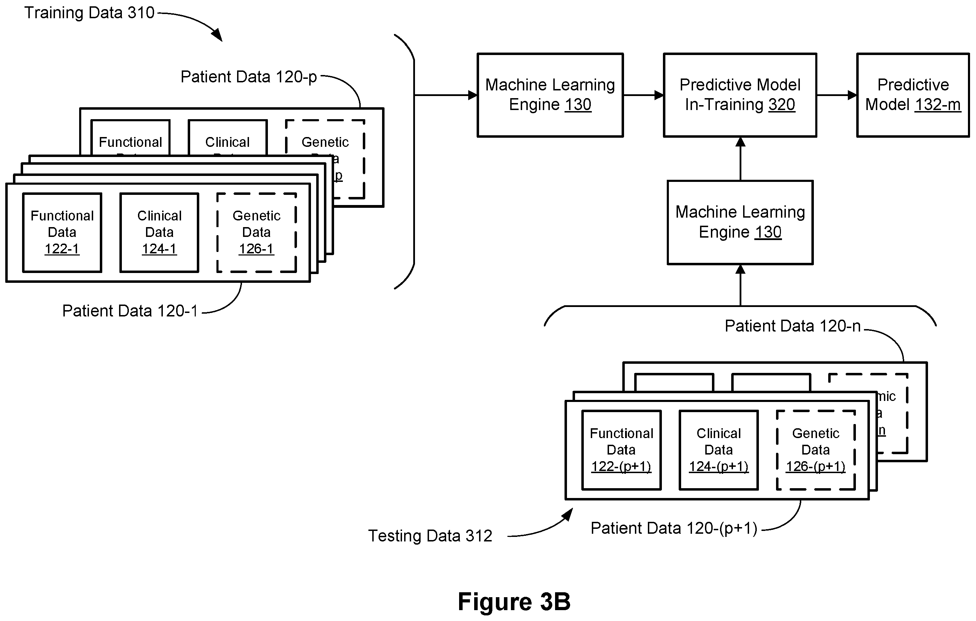

Referring to Figure 3B, the machine learning engine 130 uses the training data 310 and the testing data 312 to train the predictive model of the plurality of predictive models 132. The machine learning engine 130 uses the training data 310 to train (e.g., generate) a predictive model in-training 320 and uses the testing data 312 to test and refine the predictive model in-training 320 in order to generate (e.g., train) the predictive model 132-m. Once the predictive model 132-m is trained, the predictive model 132-m can be used to predict a patient's response to a specific drug therapy.

This process can be repeated for a plurality of predictive models 132, where the patient data 120 and the plurality of patients 110 used as inputs to train each predictive model 132 differs for each different (e.g., distinct) model.

Figures 4A - 4D illustrate examples of functional data from patients. The functional data described in Figures 4A - 4D may correspond to the functional data 122 that is used in training the predictive model(s) 132 (as shown in Figure 1A) and/or functional data 142 for a new patient 140 whose patient information is input into the trained predictive model(s) 132 to provide a prediction(s) of the new patient's response to different drug therapies (as shown in Figure 1B). The functional data includes information regarding: (i) cell viability; (ii) cell population distribution with respect to cell size, cell shape, and biomarker expression; and (iii) drug sensitivity of the cells. The functional data is collected from live cells harvested from the patient by a physician. The live cells are harvested from the diseased site (e.g., tumor site) via biopsy.

Figure 4A illustrates data on patients' cell viability, which provides information regarding the ability of cells to maintain or recover viability (e.g., ability to stay alive and/or grow). Initial cell viability 412 can be determined through the use of cell viability assays and cell proliferation assays. Initial cell viability 412 may be quantifiable between 0 and 1 (e.g., 0% and 100%), where 0 corresponds to a completely dead state and 1 corresponds to a completely alive state (e.g., 0 = all the cells are dead and 1 = all the cells are alive). Cell viability assays can also determine (e.g., measure) any of: the physical integrity of the cells (e.g., cell appearance), metabolic activity of the cells (e.g., metabolism), mechanical activity of the cells, mitotic activity of the cells, and *in-vivo* function (e.g., proliferation capacity) of a given cellular phenotype. Table 410 includes information about patients' cell viability 412. In this example, information regarding the cell viability 300 is provided for three patients who are each identified by their identification number (e.g., "Patient ID #"). Table 410 shows that:
- a first sample corresponding to patient 148 (e.g., cell samples retrieved via biopsy from a tumor site on the patient 148) has 0.87 x 10⁷ cells, and that the initial cell viability 412 of the sample is 95.4%;
- a second sample corresponding to patient 247 (e.g., cell samples retrieved via biopsy from a tumor site on patient 247 has 1.44 x 10⁷ cells, and that the initial cell viability 412 of the sample is 88.9%; and
- a third sample corresponding to patient 392 (e.g., cell samples retrieved via biopsy from a tumor site on patient 392 has 1.13 x 10⁷ cells, and that the initial cell viability 412 of the sample is 93.5%.

The number of cells 414 in the sample do not correspond to and are not indicative of the initial cell viability 412 such that there is no direct or indirect relationship between the number of cells 414 in a sample and the initial cell viability 412 of the sample. Each patient ID number corresponds to a single and unique patient such that a given patient ID is associated with only one patient and that a given patient is associated with only one patient ID.

In some implementations, the cell viability information is omitted in the case where the number of cells 414 is below a predefined threshold number of cells. For example, if the predefined threshold number of cells is 0.1 x 10⁷ cells, the initial cell viability data 412 may be considered to not be usable and the initial cell viability data may be omitted or imputed through other means.

In some implementations, the number of cells 414 and the initial cell viability 412 of a sample may vary based on the disease type (e.g., type of cancer, tumor type) and the drug therapy (e.g., chemotherapy, anti-cancer drug). For example, the number of cells 414 and the initial cell viability 412 may be several folds higher or lower than the values shown in table 410.

In some implementations, the choice of cell viability assay is based on the type of diseases (e.g., type of cancer) and/or the type of cell. For example, a first cell viability assay (e.g., assay method) is used for determining the number of cells 414 and initial cell viability 412 when the sample is obtained from lymphoma in the lungs, and a second cell viability assay (e.g., assay method) that is different from the first cell viability assay is used for determining the number of cells 414 and initial cell viability 412 when the sample is obtained from a glioblastoma in the brain.

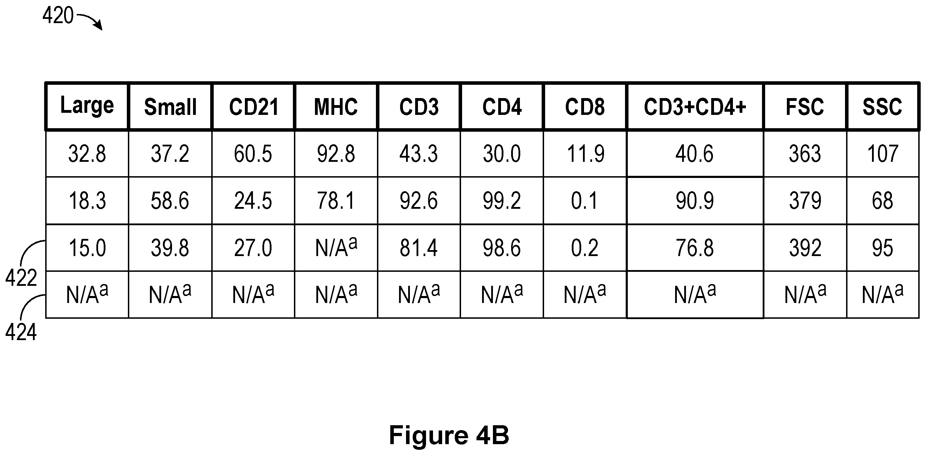

Figure 4B illustrates data on the distribution of the cell population in a sample with respect to cell size, cell shape, and biomarker expression. Data on the distribution of the cell population in a sample can be determined (e.g., measured, obtained) by using flow cytometry to analyze the sample. Flow cytometry is method for analyzing (e.g., determining, identifying, measuring, obtaining) the appearance (e.g., shape, integrity, size, volume) and the phenotypic features (e.g., biomarker expression) of cells in the sample. Table 420 illustrates an example of functional data regarding the distribution of the cell population in a sample for four different patients. Table 420 shows flow cytometry results for four patients, with each row corresponding to a different (e.g., distinct patient). Each column in the table 420 corresponds to a different characteristic, such as different phenotypic features or different biomarker expression.

In some implementations, different characteristics (e.g., cell integrity, biomarker expression, phenotypic features) and/or different number of characteristics may be identified (e.g., obtained, collected) for different patients. In some implementations, the characteristics that are collected for a specific patient may depend on (e.g., be based on) the patient's disease type (e.g., type of cancer). For example, 124 features may be collected for a lymphoma patient and 132 features may be collected for a glioblastoma patient. Some, all, or none of the 124 features collected for the lymphoma patient may overlap (e.g., be the same as) the 132 features collected for the glioblastoma patient.

In some implementations, some or all of the characteristics may not be identified for a given patient. For example, row 422 of table 420 shows that the information for the characteristic MHC is missing, and row 424 of table 420 shows that the information for the all of the characteristics are missing. Missing data (e.g., missing information) may be due to any number of reasons, such as poor sample quality, instrument error, and/or human error, for example. In some implementations, the missing data is omitted (e.g., excluded, not included). In some implementations, the missing data is imputed (e.g., inferred). The method of imputation (e.g., inference) may vary and depends on the type of data that is missing. For example, missing information corresponding to the characteristic CD4 may be imputed using a first method and missing information corresponding to the characteristic SSC may be imputed using a second method that is different from the first method. For example a method of imputing missing information includes using a k-nearest neighbors algorithm where k is a predefined integer, such as a 10-nearest neighbors algorithm.

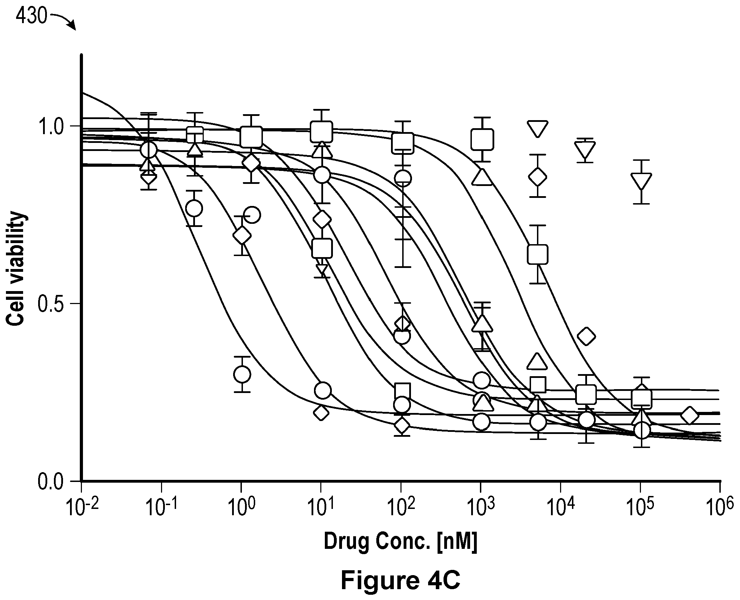

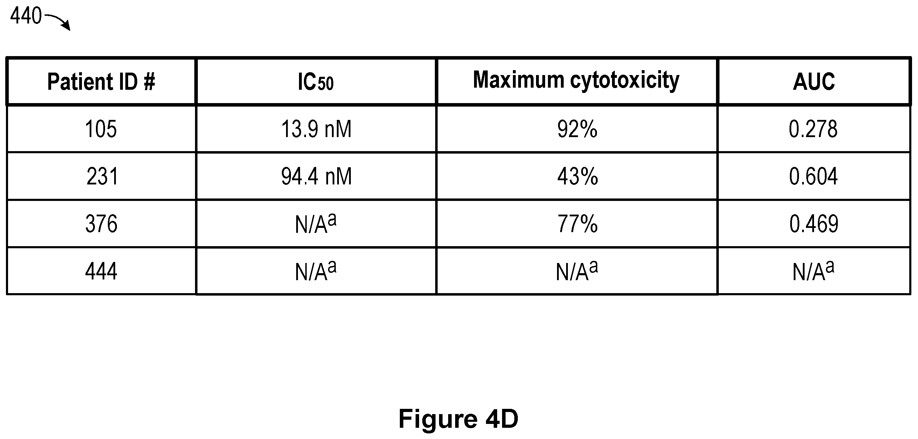

Figures 4C and 4D illustrates data on the sensitivity of the living cells in the sample to a given drug (e.g., anti-cancer drug, a chemotherapeutic drug). The drug sensitivity of the cells are determined by measuring a change in cell viability due to drug exposure. Referring to Figure 4C, graph 430 illustrates raw data collected from an assay that measures cell viability due to varying drug dosages. One or more features are extracted (e.g., obtained, calculated, measured, determined) from the collected data, including any of: IC₅₀ (which corresponds to a drug toxicity level at which cell viability decreases significantly due to drug dosage), maximum toxicity, and area under the curve (AUC). The IC₅₀ value is the drug concentration value (e.g., drug dosage value) at a mid-point of a downward slope of a plot showing decrease in cell viability with increasing drug dosage. The maximum toxicity value is a cell viability value corresponding to an asymptote of the plot. Thus, an AUC value can be estimated based on the IC₅₀ value and the maximum toxicity value. In some implementations, two different plots with a same IC₅₀ value and a same maximum toxicity value may have a different y-intercept value. Thus, in some cases the AUC value is determined (e.g., estimated or approximated) using (e.g., based on) the IC₅₀ value, the maximum toxicity value, and a baseline value (e.g., y-intercept value).

Referring to Figure 4D, table 440 illustrates an example of the drug sensitivity data for a specific drug (e.g., a first drug, a same drug) collected for four different patients.

The IC₅₀, maximum toxicity, and AUC are correlated with one another. In some implementations, such as when the number of patients is greater than a predefined threshold of patients, the AUC can be determined (e.g., calculated or estimated) based on the IC₅₀ and the maximum toxicity values. For example, when the drug sensitivity data includes information for more than 100 patients, the AUC may be calculated (e.g., via a machine learning algorithm) using the IC₅₀ and maximum toxicity values as input variables.

In some implementations, the functional data (e.g., functional data 122 of patient data 120 corresponding to a patient 110 of the plurality of patients or the functional data 142 of new patient data 141 corresponding to a new patient 140) may include the IC₅₀ value and the maximum toxicity value. In some implementations, the functional data (e.g., functional data 122 of patient data 120 corresponding to a patient 110 of the plurality of patients or the functional data 142 of new patient data 141 corresponding to a new patient 140) may include the AUC value.

The number of drug therapies (e.g., drugs and drug combinations) that are tested for each patient can vary from patient to patient. For example, sample(s) from a patient corresponding the patient ID# 105 may be tested for 2 different drugs or drug combinations, and sample(s) from a patient corresponding the patient ID# 231 may be tested for 12 different drugs or drug combinations. In some implementations, the drugs or drug combinations that are tested for given patient may correspond to (e.g., include) one or more drugs or drug combinations with which the patient is currently being treated. In some implementations, the drugs or drug combinations that are tested for a given patient may correspond to (e.g., include) one or more drugs or drug combinations that the patient is not currently taking as part of his/her treatment (e.g., may include drugs or drug combinations other than the drugs or drug combinations with which the patient is currently being treated). In some implementations, the drugs or drug combinations that are included in the drug sensitivity data for training a model to predict a patient response's to a specific drug may include drugs other than the specific drug and/or may include drug combinations that do not include the specific drug. For example, drug sensitivity data (e.g., value(s) for any or all of the IC₅₀, maximum toxicity, and AUC) for the drug cyclophosphamide may be used as part of the input when training a model to predict a lymphoma patient's response to the drug lomustine.

Figure 3F illustrates an example of clinical data from patients. The clinical data described in Figure 3F may correspond to clinical data 124 that is used in training the predictive model(s) 132 (as shown in Figure 1A) and/or clinical data 144 for a new patient 140 whose patient information is input into the trained predictive model(s) 132 to provide a prediction(s) of the new patient's response to different drug therapies (as shown in Figure 1B). The clinical data is provided by either the patient or the patient's physician. In some implementations, the clinical data is extracted from one of more patient information documents (e.g., medical charts, doctor's notes) via one or more processing methods such as natural language processing or optical character recognition (OCR). Table 510 illustrates an example of clinical data received for five different patients, each row of the table 510 corresponding to a different (e.g., distinct) patient. The rows in table 510 are examples of some different metrics or features that are included in the clinical data. For example, the "Chemo" column 512 indicates the drug therapy (e.g., chemotherapy) with which the patient is currently treated, and the response column 514 corresponds to the patient's response to the drug therapy identified in column 514 In this example, the patient's response in the response column 514 is coded into four possible responses based on the Response Evaluation Criteria in Solid Tumors (RECIST) scoring system: (i) "CR" denoting clinical remission, (ii) "PR" denoting partial remission, (iii) "SD" denoting stable disease, and (iv) "PD" denoting progressive disease. The patient's response in the response column 514 is calculated based on information that is provided by the patient's physician. For example, the patient's response is recorded (using one of the 4 codes) by a physician at each patient visit, resulting a plurality of recorded responses over time (in some cases, over a period of up to a few years). Using the plurality of recorded responses for the patient, a net response is calculated using equation (1), shown below. $Response = max \left(\left\{{r}_{CR}, {r}_{PR}, {r}_{SD}, {r}_{PD}\right\}\right)$

Each response category weight (e.g., *r_{CR}*, *r_{PR}, r_{SD}, r_{PD}*), denoted below as *rₓₓ,* is determined by equation (2), shown below. ${r}_{xx} = {\sum }_{i = 0}^{n} 1 - \frac{\left|{t}_{i}-a\right|}{b}$

The number of weekly responses for a given drug that a patient is currently taking as part of his or her drug therapy is denoted in equation (2) as n and *tᵢ* is the elapsed time between the sample date and the response *i.* In some implementations, the variables *a* and *b* can be adjusted on a per-model basis. In some implementations, the variables *a* and *b are* determined based on criteria based on the disease of interest (e.g., type of cancer), and the drug therapy of interest (e.g., drug therapy 150). For instance, for initial modeling for lymphoma, *a =* 4 and *b* = 76. In contrast, for modeling for adenocarcinoma, *a =* 3 and *b* = 78. In some implementations, the variables *a* and *b* are adjusted over time based on treatment regimens and disease time courses. For example, if many patients die in the first 90 days, variable b may be reduced after the first 90 days. In another example, if complete responses take much longer to develop for a specific cancer type (e.g., a slow progressing type of cancer), the variable b may be larger compared to the variable b for a model for a different type of cancer that is faster progressing.

For example, using equations (1) and (2), the response of a patient that shows complete response after a single dose of cyclophosphamide immediately after sampling is weighted as a "more confident" response (e.g., has a higher weight) than the response of a patient who achieves complete response after 3 weeks of intermittent cyclophosphamide therapy, as in CHOP. In another example, the response of a patient who maintains the same response over several weeks of doxorubicin alone would be weighted as a 'more confident' (e.g., has a higher weight) than the response of a patient who received doxorubicin once or twice over a period of months.

In some implementations, the clinical data includes information regarding the concentration of different biochemicals in blood. For example, column 516 of table 510 shows the total protein concentration. In some implementations, the clinical data may include information the concentration of any number of biochemicals, such as 1, 2, 5, 20, 25, or 29 different biochemicals.

In some implementations, the clinical data includes information regarding addition medication (e.g., non-chemotherapeutic drugs) that the patient is also taking during chemotherapy. In some implementations, information regarding addition medication is provided as a binary value (e.g., "yes" denoting taking other medication, or "no" denoting not taking other medication). In some implementations, information regarding addition medication identifies any additional medication that the patient is taking during chemotherapy. In some implementations, information regarding addition medication also identifies a frequency and/or a dosage of the addition medication.

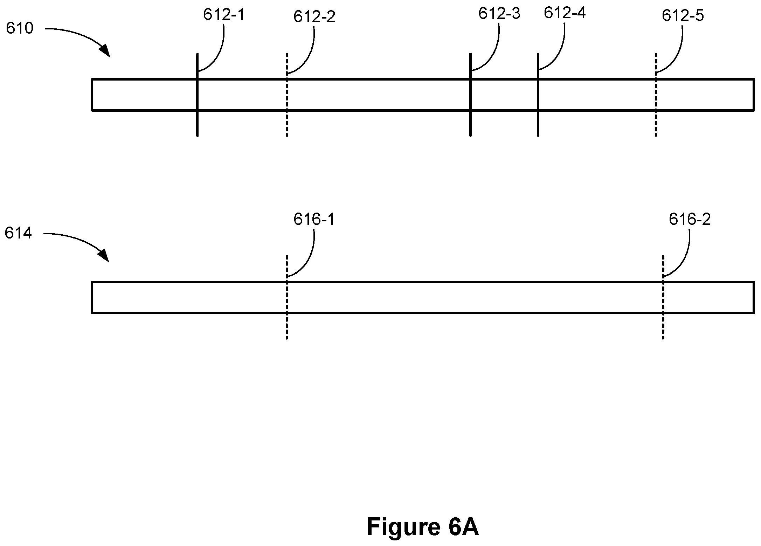

Figures 6A - 6D illustrate examples of genetic data from patients. The genetic data described in Figures 6A - 6D may correspond to genetic data 126 that is used in training the predictive model(s) 132 (as shown in Figure 1A) and/or genetic data 146 for a new patient 140 whose patient information is input into the trained predictive model(s) 132 to provide a prediction(s) of the new patient's response to different drug therapies (as shown in Figure 1B). The genetic data includes genetic mutation data that is obtained from live cells that are collected by a physician via biopsy. The genetic data includes DNA and RNA extracted (e.g., sequenced) from the live cells. In some implementations, the live cells include cells that are harvested from a diseased site (e.g., tumor site). In some implementations, the live cells include a paired sample that includes cancerous cells that are harvested from a diseased site (e.g., somatic cells harvested from a tumor site) and healthy cells that are harvest from a healthy site (e.g., germline cells harvested from a non-cancerous site). The type of live cells harvested (e.g., the location of the healthy sites) may vary from patient to patient and in some cases, is determined based at least on the disease type (e.g., type of cancer). In some implementations, the healthy cells that are harvested from a healthy site (e.g., germline cells harvested from a non-cancerous site) may be harvested via non-invasive or minimally invasive techniques, such as a cheek swab.

Tumor development in an individual can be influenced by a combination of variants in both non-reproductive cells and reproductive cells. In some implementations, tumor development can be influenced by a combination of variants that are either inherited from a parent, in which case the variant would be a germline variant (e.g., a reproductive cell variant), or are developed after birth, in which case the variant would be a somatic variant. The somatic variants are not passed down generation to generation since they are not present in reproductive cells. In some cases, a single somatic variant (e.g., somatic mutation) is sufficient to cause a tumor, but more commonly, multiple variants are required for a tumor to develop. Since mutations slowly accumulate in cells over time and there are many robust ways for the body to remove aberrant cells, tumor development is typically a slow process. However, certain germline variants can greatly accelerate this process. For example, the presence of BRCA1 and BRCA2 mutations in an individual greatly increase risk of breast and ovarian cancer. This is because the BRCA1 and BRCA2 genes aid in DNA damage repair and function as tumor suppressors. If they are nonfunctional, other DNA damage repair processes can continue to function normally, but their efficiency in tumor suppression is reduced, thus greatly increasing the risk of cancer.

The ability to distinguish between germline variants (e.g., germline mutation) and somatic variants (e.g., somatic mutation) can be useful, especially when used in conjunction (e.g., combination) with other tumor-specific assays. For example, a variant in a liver enzyme may affect the patient's ability to process a drug, thereby doubling or even tripling the time that drug is active in the patient's body and leading to a higher risk of the patient experiencing side effects. However, if this variant is a somatic variant that is not expressed or useful in a cancerous cell (e.g., a lymphoma cell), it can be safely ignored if this variant is present only in cancerous cells of the tumor and not in other healthy cells. Thus, by including information obtained from both diseased cells (e.g., cancerous cells biopsied from a tumor site) and healthy cells (e.g., non-cancerous cells obtained from a healthy site) for each patient 110 of the plurality of patients and for the new patient 140, certain associations between germline variants and somatic variants to the patient's response to a specific drug therapy can be used in the one or more predictive models 132 to predict the patient's response (e.g., response of patient 140) to a specific drug therapy.

Figure 6A provides an example of a variants in cancerous cells and non-cancerous cells according to some implementations. In this example, variants 612-1 to 612-5 are found in a cancerous cell sample 610 and variants 616-1 and 616-2 are found in a non-cancerous cell sample 614 of a same patient (e.g., patient 110 or new patient 140). Variant 612-2 in the cancerous cell sample 610 and variant 616-1 in the non-cancerous cell sample 614 correspond to one another (e.g., correspond to a same variant, are a same variant within a gene), and variant 612-5 in the cancerous cell sample 610 and variant 616-2 in the non-cancerous cell sample 614 correspond to one another (e.g., correspond to a same variant, are a same variant within a gene). Variants 612-1 and 612-5 are known to be common in the population (e.g., known to be common in humans, known to be common in humans of European descent, known to be common in dogs, known to be common in Dalmatians), variant 612-2 is an uncommon variant in the population, variant 612-3 is a newly identified variant, and variant 612-4 is known to be a common tumor variant. Using information obtained from both the cancerous cell sample 610 non-cancerous cell sample 614, variants 612-2 and 612-5 can be identified as germline variants since they are present in both the cancerous cell sample 610 and the non-cancerous cell sample 614 (labeled as variants 616-1 and 616-2 in the non-cancerous cell sample 614). In contrast, variants 612-1, 612-3, and 612-4 are identified as somatic variants since they are present in the cancerous cell sample 610 and are not present in the non-cancerous cell sample 614.

In some implementations, paired samples of a patient 110 or a new patient 140 that include cells from a diseased site and a healthy site (e.g., paired tumor and normal samples) are not available, one or more other methods may be used to determine (e.g., estimate, guess) whether variant is a germline variant or a somatic variant. In some implementations, the likelihood of a variant being germline variant or somatic variant can be determined (e.g., guessed, estimated) based on the frequency of the variant in a population. For example, variants that are very common in a population (e.g., known to be common in a specific dog breed) are likely to be a germline variant that is present in that specific population (e.g., present in that specific dog breed).

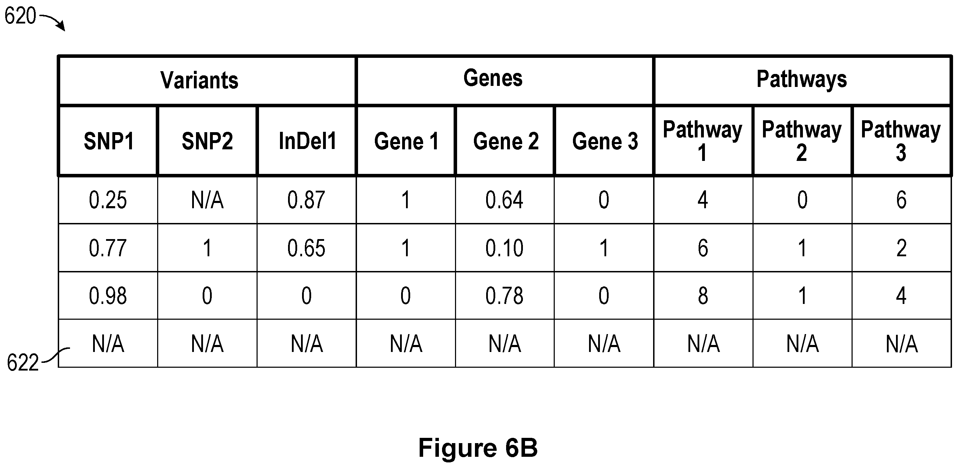

Referring to Figure 6B, table 620 shows an example of a genetic data received for four different patients, where each row of the table 620 corresponds to a different (e.g., distinct) patient. The extracted DNA is sequenced to generate raw DNA sequencing reads. Bioinformatics analysis on the raw DNA sequencing reads is used to identify any relevant genetic variants for the disease of interest (e.g., the type of cancer of interest) and the prevalence of the identified relevant genetic variants. In some implementations, a subset, less than all, of the extracted DNA is sequenced. The subset of the extracted DNA includes portions of the DNA that have previously been identified as being important in tumor development, drug response, and/or treatment resistance. In some implementations, the genetic data is encoded as a binary value corresponding to the presence or absence of mutations, a variant allele frequency, or a number of variants. The genetic data may be assessed at the level of genetic coordinates, genes of interest, or pathways of interest. Any number of genes can be sequenced in order to generate the genetic data. For example, the number of genes included in the genetic data may be at least 100 genes, 1,000 genes, 10,000 genes, or more. In some implementations, the sequencing panel of genes include genes that have been previously identified as being relevant to (e.g., implicated in, involved in, associated with) oncogenesis, treatment response (e.g., response to chemotherapy, response to anti-cancer drug(s)), and/or relapse in human or canine (as determined in medical and scientific studies). The genes and the number of genes can be expanded or altered based on any of: performance of the predictive model(s) 132, current literature (e.g., scientific literature, medical literature), and bulk genetic sequencing of some or all genes (e.g., whole genome) or exons (e.g., the whole exome).

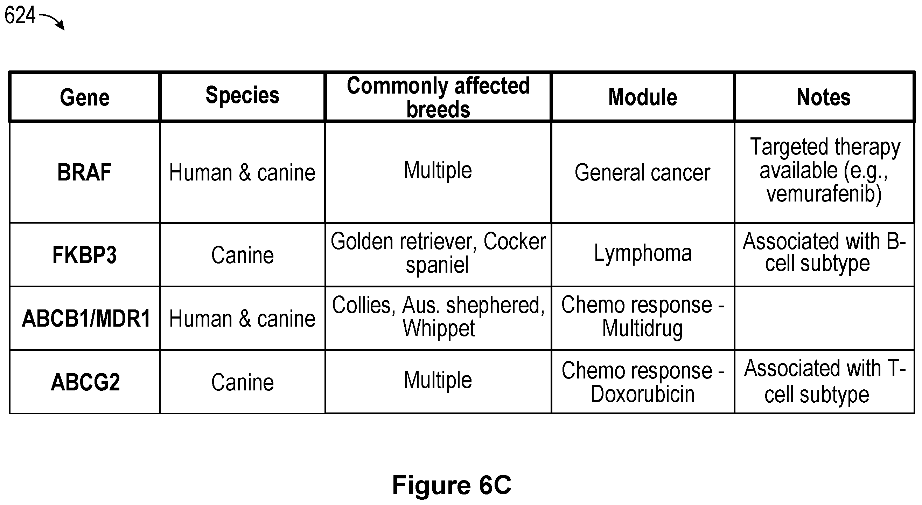

Figure 6C provides an example of genes included in the genetic data in accordance with some implementations. In some implementations, genes included in a gene panel selected based on literature and database review of the effects of various genes in (e.g., associated with, involved in) studies of specific cancer types and in chemotherapy response. For example, a gene panel for generating genetic data 126 (corresponding to a patient 110 for training one or more predictive models 132) of genetic data 146 (corresponding to a new patient 140 whose patient data 141 is input in the one or more trained predictive models 132 for predicting the new patient's response to one or more drug therapies) corresponding to a predictive model 132 associated with canine lymphoma and drug therapies used for treating canine lymphoma may include genes that have been identified in both human and canine studies of both canine lymphoma and chemotherapy response.

The gene sequencing panels are modular, and different modules represent gene groups such as general tumor-relevant genes, disease-specific genes (e.g., important for lymphoma specifically, or specifically important for carcinoma), and genes related to both general chemotherapy efficacy or efficacy of specific drugs. Genes that are related to general chemotherapy efficacy and/or efficacy of specific drugs are especially important for testing response to targeted therapies that may specifically target one or a small number of genes. In some implementations, such as in veterinary applications, some genes may only be relevant for a certain breed that is well known to harbor a particular mutation. However, it is not cost effective to customize sequencing panels on a per-individual basis, so panels are applied in a species- and disease-specific manner (e.g., a canine lymphoma panel or human osteosarcoma panel) and include drug-specific modules based on most common therapies for a given cancer type. Modules can vary in size from a few dozen genes to several hundred genes. As an example panel, the canine lymphoma panel currently consists of 234 genes. Table 624 provides examples of genes that may be included in gene panels.

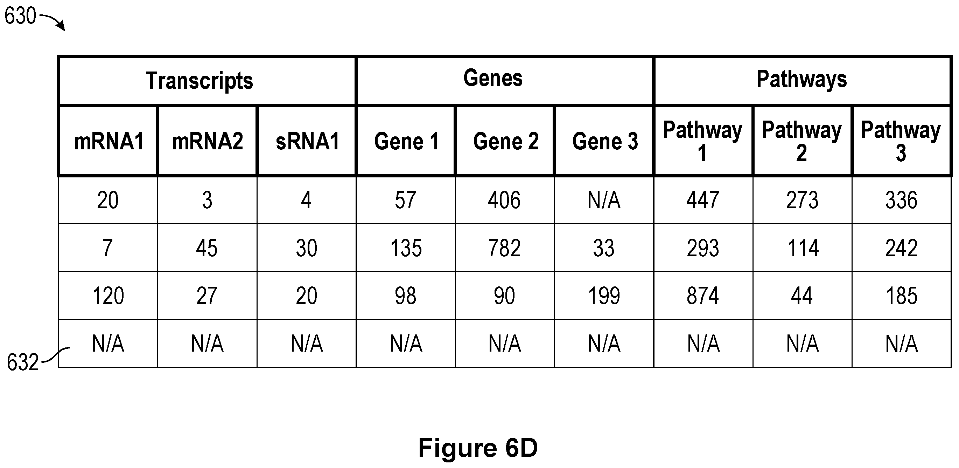

Referring to Figure 6D, table 630 shows an example of a genetic data received for four different patients, where each row of the table 630 corresponds to a different (e.g., distinct) patient. The extracted RNA is sequenced to generate raw RNA sequencing reads. Bioinformatics analysis on the raw RNA sequencing reads is used to identify RNA expression levels, which may include any of mRNA and sRNAs of interest for a specific disease (e.g., a specific type of cancer). Thus, when obtaining RNA information for different diseases, the specific mRNAs and sRNAs that are in the genetic data may vary from disease to diseases. For example, the genetic data may include a first set of mRNAs and sRNAs when using genetic data to train a predictive model 132 to predict patient response to lymphoma, and the genetic data may include a second set of mRNAs and sRNAs, that is different from the first set of mRNAs and sRNAs, when using genetic data to train a predictive model 132 to predict patient response to melanoma. The second set of mRNAs and sRNAs differs from the first set of mRNAs and sRNAs by one or more of mRNA or sRNA. For example, the second set of mRNAs and sRNAs may include an mRNA or sRNA that is not included in the first set of mRNAs and sRNAs.

The RNA information is encoded using one or more normalization strategies that can be assessed at the transcript, gene, or pathway level. For example, table 630 shows an example of RNA data for four different patients (each patient corresponding to a row). While data shown in table 630 is normalized using transcripts per million, other methods such as log fold change from normal tissue can also be used to normalize the data.

In some implementations, the RNA data is expressed as a ray number of reads (e.g., raw data before normalization). In some implementations, the RNA data is expressed as a measure of relative abundance of a given transcript (e.g., normalized data). This data can be critical independent of mutation data. For example, a mutation that isn't in the sequencing panel may affect the expression level of an important gene that isn't mutated in the patient (e.g., patient 110 or new patient 140). Thus, even though genetic sequencing results appear "normal" for a specific gene, it may be expressed at quadruple normal levels or not expressed at all in cancerous cells in a tumor. Furthermore, if a particular gene is poorly characterized or not included in the sequencing panel, extreme over-expression may indicate that the particular gene is important in a given tumor or may indicate over-activity of a pathway that can be effectively targeted by chemotherapy. Conversely, gene expression can also be an important corollary to genomic sequencing. A gene may have a critical mutation that is very important in a particular type of cancer (e.g., carcinoma, lymphoma), but if other processes in the cancerous cells (e.g., in the tumor) cause the gene to be under-expressed, drugs targeting the gene or mutation may be less effective than in other similar tumors. While the genetic data 126 obtained from DNA extracted from the sample may provide an indication of mutations within the genes that may be associated with the cancer, genetic data 126 obtained from RNA extracted from the sample can provide an indication of what genes are being expressed in the cancerous cells and thus, provide insight regarding which drug therapies may be effective based on the association between specific pathways and specific drug therapies. Thus, by including genetic data 126 obtained from both DNA and RNA extracted from the patient's cell samples, the one or more predictive models 132 are able to form associations between genes and diseases as well as pathways and drug therapies, allowing the one or more predictive models 132 to provide robust prediction results 151.

In some implementations, some or all of the genetic data may not be identified for a given patient. For example, row 622 of table 620 shows that the genetic DNA information for that patient is missing. Similarly, row 632 of table 630 shows show that the genetic RNA information for that patient is missing. Missing data (e.g., missing information) may be due to any number of reasons, such as poor sample quality, lack of sufficient sequencing quality and/or depth, and/or technical difficulties associated with sample isolation and sequencing library preparation, for example. In some implementations, the missing data is omitted (e.g., excluded, not included). In some implementations, the missing data is imputed (e.g., inferred). The method of imputation (e.g., inference) may vary and depends on the type of data that is missing. For example, missing information corresponding to a gene (such as Gene 1) may be imputed using a first method and missing information corresponding to a different gene (e.g., Gene 2) may be imputed using a second method that is different from the first method. In another example, missing information corresponding to a gene (such as Gene 1) may be imputed using a first method and missing information corresponding to a pathway or variant (such as Pathway 1 or SNP2) may be imputed using another method that is different from the first method.

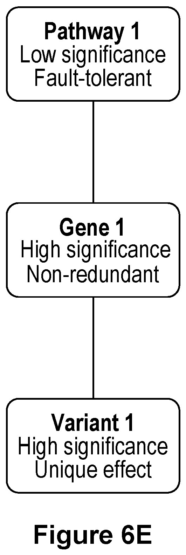

Figures 6E - 6G illustrate examples of how pathways, genes, and variants can correspond to one another in accordance with some implementations. Using a unique combination of variants, genes, and pathways in a given model allows the one or more predictive models 132 to be tailored to increase (and ideally, maximize) predictive efficacy. The progression of variant to gene to pathway can be thought of as a hierarchy to control the scale at which a given biological unit is modeled (e.g., is included in the model). Pathways can contain many genes, and each gene can contain many variants. Figures 6E - 6G illustrate how variants, genes, and pathways can affect tumor behavior and response to chemotherapy, with the colors representing the level of predictive value our model can gain from a particular entity.

In some cases, as shown in Figure 6E, a single variant (e.g., Variant 1) can significantly disrupt an important gene (e.g., Gene 1) with non-redundant functions, but the single gene (e.g., Gene 1) may be part of a large pathway (e.g., Pathway 1) that is not typically important in a particular type of cancer. If the gene (e.g., Gene 1) is small and only has one important variant (e.g., Variant 1), either the gene (e.g., Gene 1) or the variant (e.g., Variant 1) can be assessed since the effects of the gene (e.g., Gene 1) and the variant (e.g., Variant 1) are highly correlated.

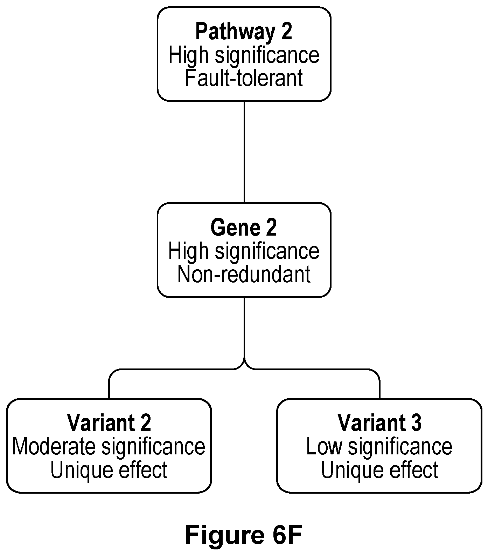

In some cases, as shown in Figure 6F, individual variants (e.g., Variant 2 and Variant 3) in a gene (e.g., Gene 2) may have unique effects of minor to moderate significance alone (e.g., many different variants may only partially disrupt a gene's function), but because the gene (e.g., Gene 2) is of high significance with a non-redundant function, any partial disruption is can inform our predictions. Despite the gene's importance (e.g., the importance of Gene 2), it may exist in a pathway (e.g., Pathway 2) where many connections are redundant, and thus most of the genes (other than Gene 2) are not particularly significant on their own.

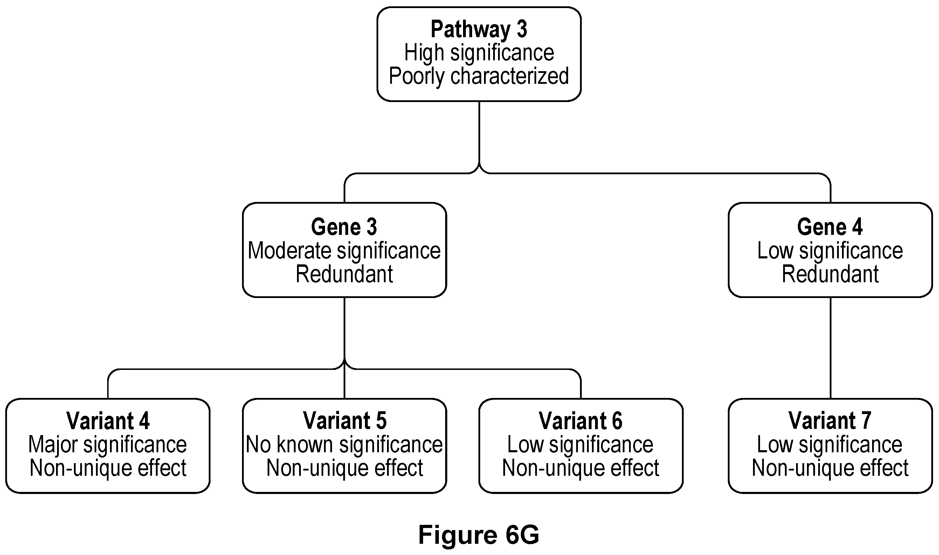

In some cases, as shown in Figure 6G, a gene (Gene 3) includes many potential variants of varying importance and characterization. The gene (Gene 3) itself is only marginally important because other genes (such as Gene 4) have similar functions. However, the pathway (e.g., Pathway 3) may represent a pathway that is fault tolerant until it reaches a critical level of disruption, at which point the pathway (e.g., Pathway 3) catastrophically fails. While all of the components of the pathway (e.g., Pathway 3) may not be well characterized, the importance of the pathway (e.g., Pathway 3) is still significant in the one or more predictive models 132 despite having incomplete information (e.g., not having complete information).

A similar conceptual model can be applied to gene expression data, but instead of variants, one would use transcripts. Each gene can produce one or multiple transcripts that can vary significantly or be highly homogeneous. The scale of the values reported by gene expression assays is very different from that reported by genomic sequencing (for example, gene expression can be encoded as "gene-specific transcripts per million total transcripts in an experiment" (TPM) versus genomic data being encoded as "presence/absence of variant" or "percent of sequencing reads in a sample with a particular variant (variant frequency)". However, despite these differences in numerical readouts for an assay, in some implementations, the general hierarchy and its application to including variables in a predictive model 132 can be very similar.

Thus, by including variants, transcripts, genes, and pathways for each patient 110 of the plurality of patients, and for the new patient 140, associations between a patient's drug response and the variants, transcripts, genes, and pathways expressed in the patient can be discerned at a higher level of detail compared to a model that utilizes only a subset of these factors. The one or more predictive models 132 can use these detailed associations and relationships between variants, transcripts, genes, and pathways to predict the patient's response (e.g., response of patient 140) to a specific drug therapy.

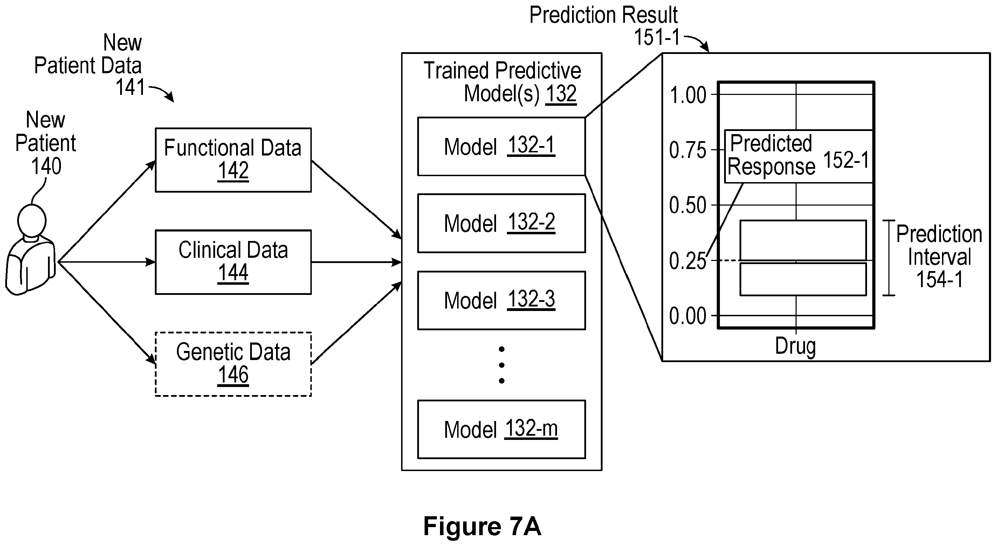

Figure 7A illustrates using predictive models 132 for predicting a patient's response to one or more drug therapies. New patient data 141 corresponding to the new patient 140 is input into the one or more trained predictive models 132. The new patient data 141 includes functional data 142, clinical data 144, and in some implementations, the genetic data 146 corresponding to the new patient 140. Each model (e.g., model 132-1, 132-2, 132-3, ..., 132-m) of the one or more trained predictive models 132 outputs a predicted response 152 and a prediction interval 154 of a predicted response. For example, the first model 132-1 outputs a first prediction result 151-1 that includes a first predicted response 152-1 and a prediction interval 154-1 of the first predicted response 152-1 corresponding to a first drug therapy. For example, the prediction interval 154-1 of the first predicted response 152-1 may be a 95% prediction interval. In the examples shown in Figured 7A and 7B, the prediction interval 154-1 of the first predicted response 152-1 is a 95% prediction interval.

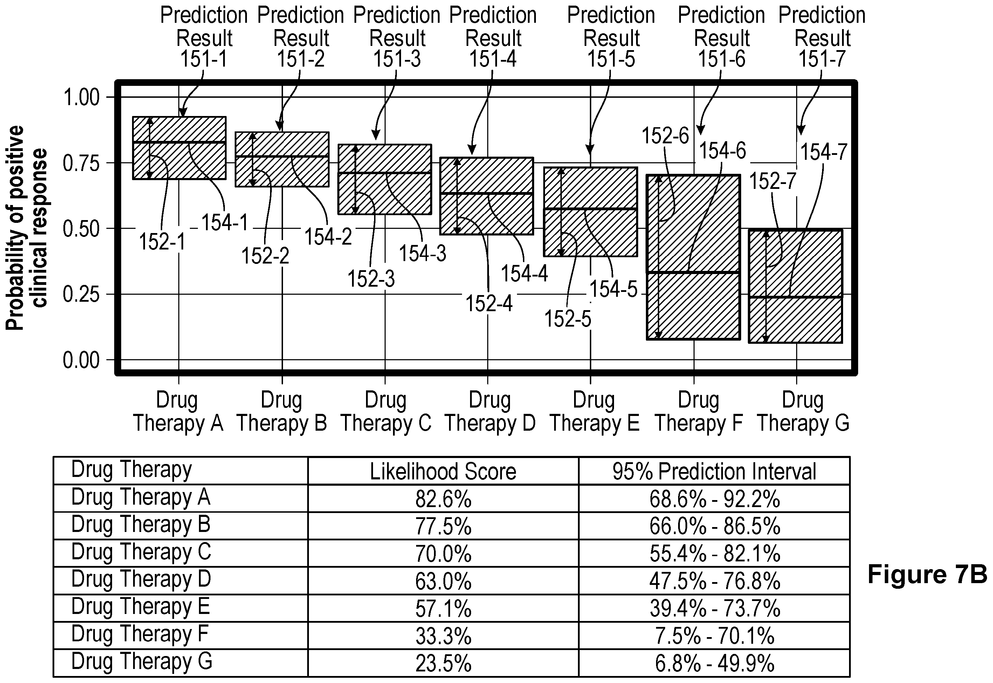

Figure 7B illustrates an example of prediction results 151 output from one or more predictive models 132. In this example, the one or more predictive models 132 outputs prediction results 151 for seven different drug therapies. Each of these drug therapies is different from each other. For example, the first drug therapy may include only a first drug; the second drug therapy may include only a second drug different from the first drug; the third drug therapy may include a first predefined combination of drugs that includes the first drug, a third drug, and a fourth drug; the fourth drug therapy may include a second predefined combination of drugs that includes the first drug and the second drug; and the fifth drug therapy may include a third predefined combination of drugs that includes the second drug and the third drug, and so on and so forth.

The prediction results 151 (e.g., prediction results 151-1 to 151-7) shown in Figure 4B indicate the likelihood (e.g., the predicted patient response 152) that the new patient 140 will have a positive response to a drug therapy, and a 95% prediction interval 154 corresponding to the predicted patient response 152. The first prediction result 151-1 indicates that the new patient 140 to whom these prediction results 151 correspond has a 82.6% chance (e.g., predicted response 152-1 of 82.6%) of having a positive response to drug therapy A, and the 95% prediction interval 154-1 between 68.6% and 92.2%. The one or more predictive models 132 also predict that the new patient 140 is 77.5% likely (e.g., predicted response 152-2 of 77.5%) to have a positive response to drug therapy B, and the 95% prediction interval 154-2 for this prediction is between 66.0% and 86.5%.

In some implementations, as shown in Figure 4B, the prediction results 151 are also color-coded to indicate the degree to which the patient is predicted to have a positive response to the drug therapy. For example, the prediction results 151-1 for drug therapy A is color coded in blue to indicate that the patient has a high likelihood of having a positive response to drug therapy A compared to the other drug therapies (e.g., drug therapies B to E) for which the one or more predictive models 132 provide predictions. In contrast, the prediction results 151-7 for drug therapy G is color coded in orange to indicate that the patient has a low likelihood of having a positive response to drug therapy G compared to the other drug therapies (e.g., drug therapies A to F) for which the one or more predictive models 132 provide predictions.

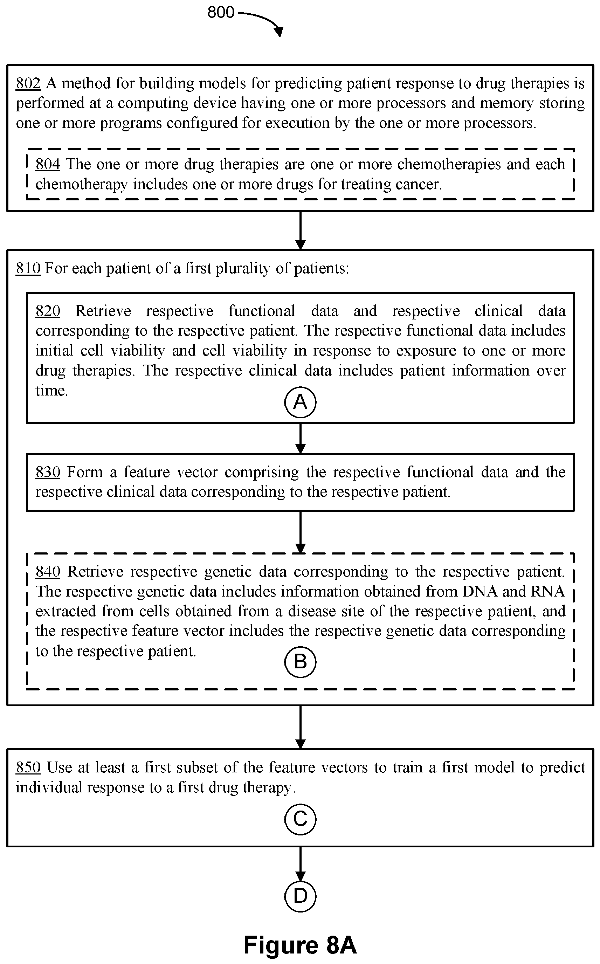

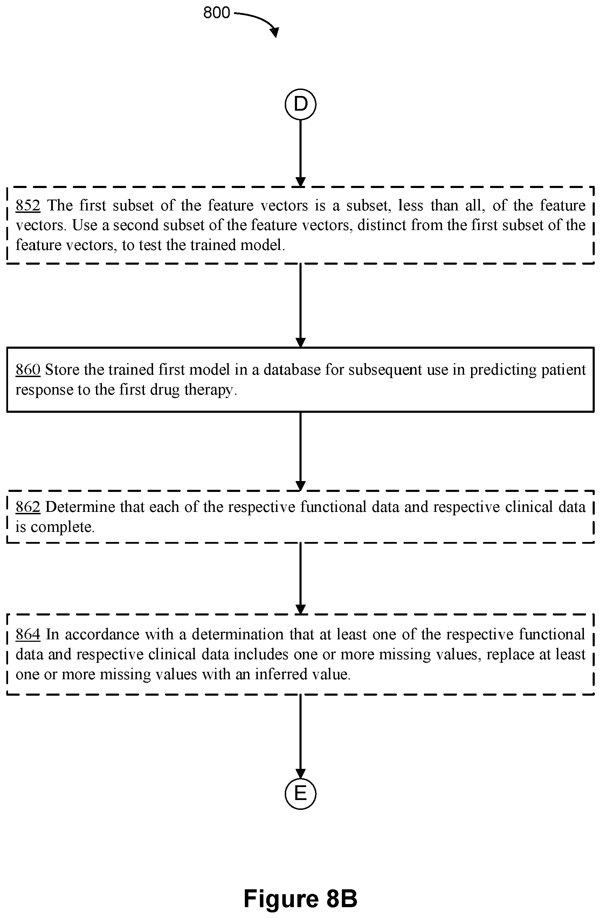

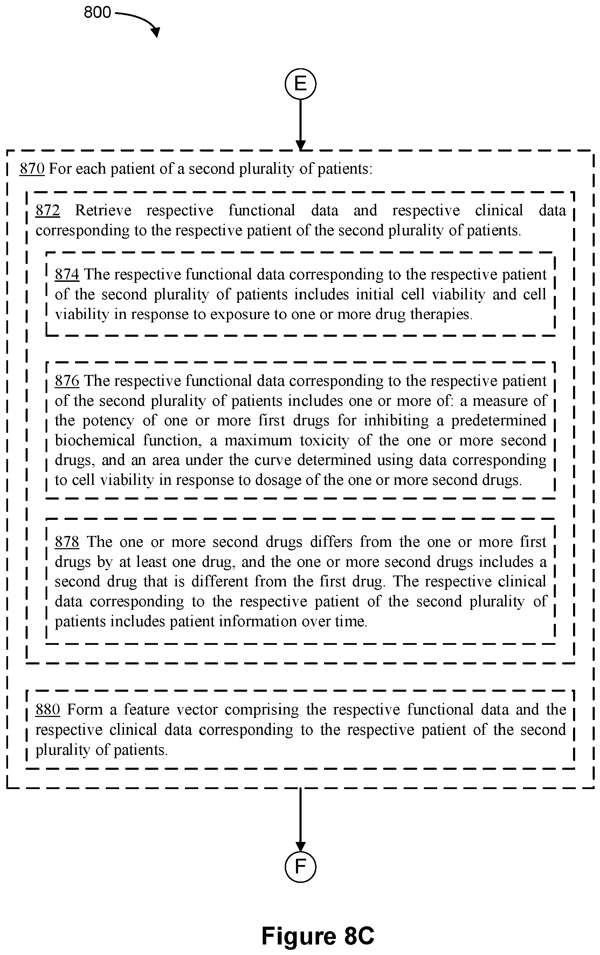

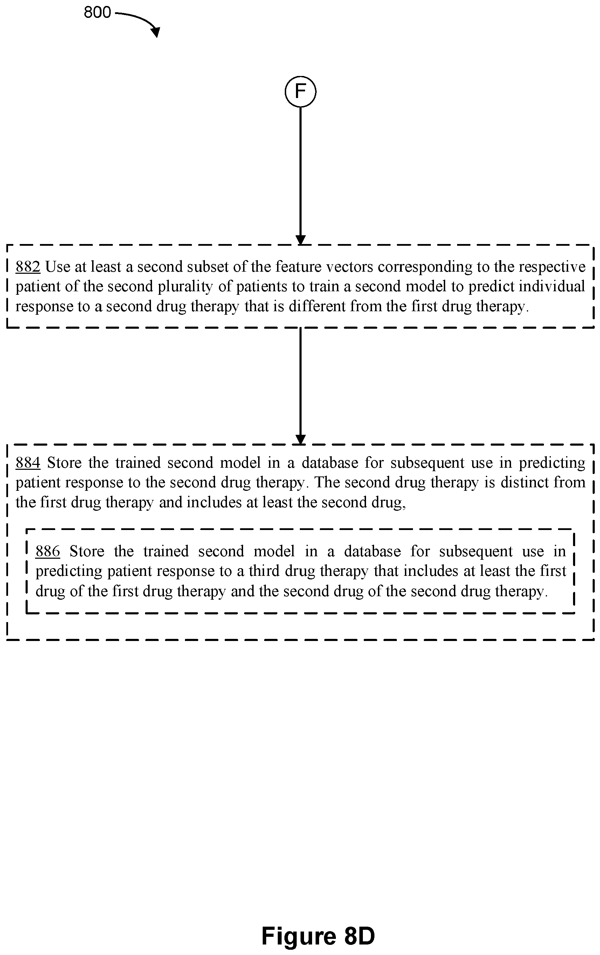

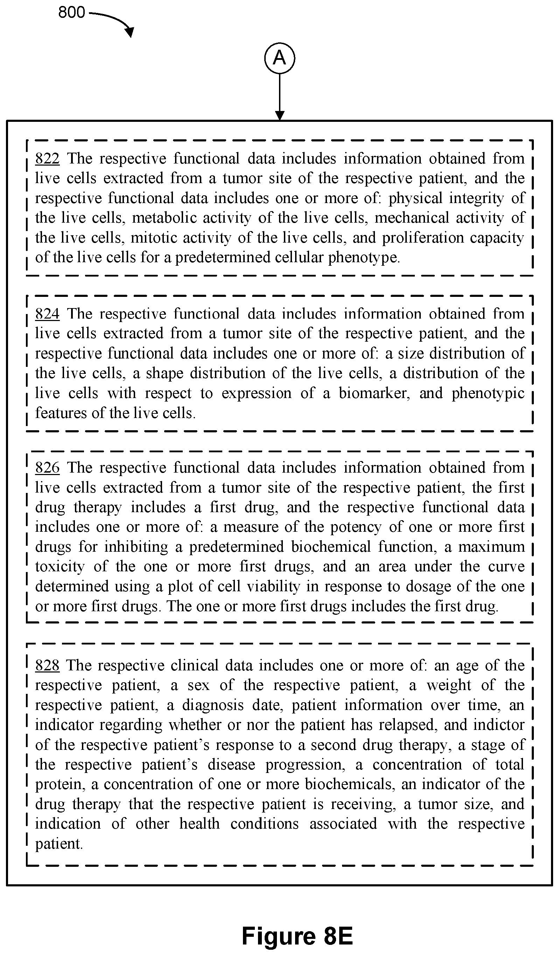

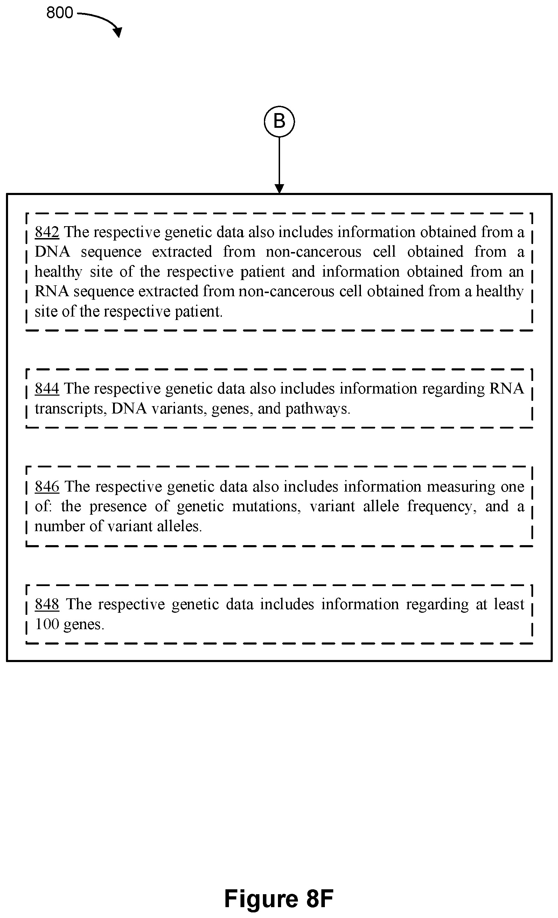

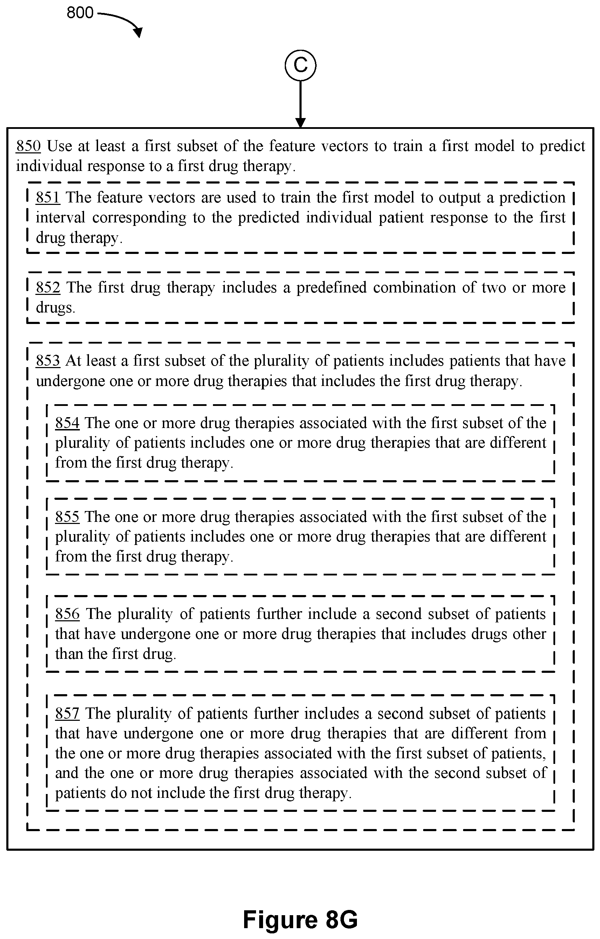

Figures 8A - 8G provide a flow diagram of a method 800 for building predictive model(s) 132 for predicting patient response 152 to drug therapies according to some implementations. The steps of the method 800 may be performed by a computer system, corresponding to a computer device 200 or a server 250. In some implementations, the computer includes one or more processors and memory. Figures 8A - 8G correspond to instructions stored in computer memory or a computer-readable storage medium (e.g., the memory 206 of the computing device 200). The memory stores (802) one or more programs configured for execution by the one or more processors. For example, the operations of the method 800 are performed, at least in part, by a machine learning engine 130.

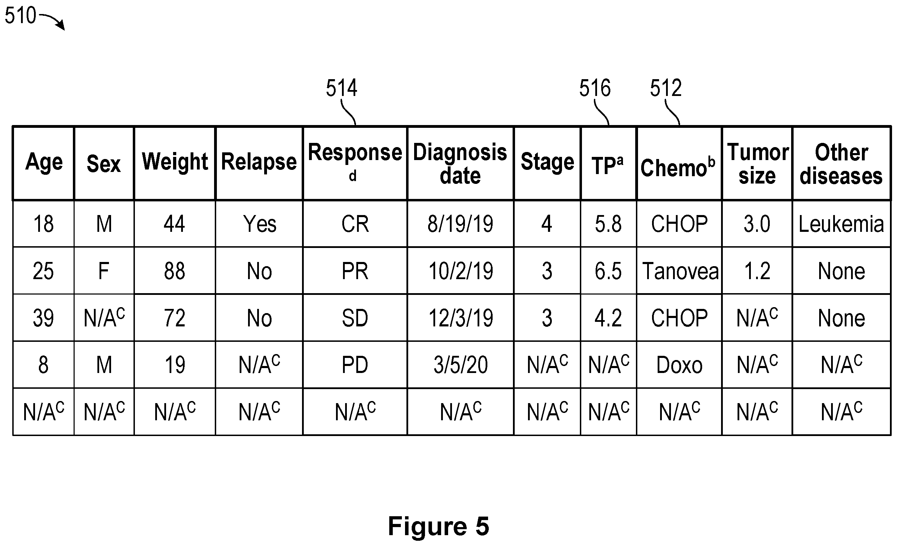

In accordance with some implementations, a computer system, computing device 200, or a server 250 performs (810) a series of operations for a plurality of patients 110 (e.g., patients 110-1 through 110-n). The system retrieves (820) respective functional data 122 and respective clinical data 124 corresponding to the respective patient. For example, as shown in Figure 1A, the computer system receives patient data 120 corresponding to a plurality of patients 110. Patient data 120 corresponding to a specific patient 110 includes respective functional data 122 and respective clinical data 124 corresponding to the respective patient 110. The respective functional data 122 includes initial cell viability 412 and cell viability in response to exposure to one or more drug therapies (e.g., drug sensitivity, illustrated by graph 430), and the respective clinical data 124 includes patient information over time (e.g., response 514 to chemotherapy). Additional details regarding the functional data 122 and clinical data 124 are provided with respect to Figures 4A - 4D and Figure 5, respectively. For each of the patients, the device forms (830) a respective feature vector that includes the respective functional data 122 and the respective clinical data 124 corresponding to the respective patient 110. The device then uses (850) at least a first subset (e.g., training data 310) of the feature vectors to train a first model (e.g., a predictive model 132-m) to predict individual patient response 152 to a first drug therapy (e.g., drug therapy 150). For example, as shown in Figure 3A, the computer system trains the first model using the training data 310 and the training data 310 is a subset, less than all, of the patient data 120. The device then stores (860) the trained first model (e.g., predictive model 132-m) in a database (e.g., database 240, 290) for subsequent use in predicting patient response 152 (e.g., patient response 152-1) to the first drug therapy.

In some implementations, for each patient of the first plurality of patients, the device retrieves (840) respective genetic data 126 corresponding to the respective patient 110. The respective genetic data 126 includes information obtained from deoxyribonucleic acid (DNA) and ribonucleic acid (RNA) extracted from cells obtained from a diseased site (e.g., tumor site) of the respective patient 110. The respective feature vector further includes the respective genetic data 126 corresponding to the respective patient 110.

In some implementations, the respective genetic data 126 also includes (842) information obtained from a DNA sequence extracted from non-cancerous cells (e.g., healthy cells) obtained from a healthy site (e.g., non-tumor site) of the respective patient 110 and information obtained from an RNA sequence extracted from non-cancerous cells (e.g., healthy cells) obtained from a healthy site (e.g., non-tumor site) of the respective patient.

In some implementations, the respective genetic data 126 also includes (844) information regarding: RNA transcripts, DNA variants, genes, and pathways. An example of genetic data 126 is provided with respect to Figures 6B and 6C.

In some implementations, the respective genetic data 126 also includes (846) information measuring one or more of: the presence of genetic mutations, variant allele frequency, and a number of variant alleles. Additional detail regarding genetic data 126 is provided with respect to Figures 6A - 6G.

In some implementations, the respective genetic data 126 includes (848) information regarding at least 100 genes, 1,000 genes, or 10,000 genes.

In some implementations, the respective functional data 122 includes (822) information obtained from live cells extracted from a tumor site (e.g., cancerous cells extracted from a diseased site) of the respective patient 110, and the respective functional data 122 includes one or more of: physical integrity of the live cells, metabolic activity of the live cells, mechanical activity of the live cells, mitotic activity of the live cells, and proliferation capacity of the live cells for a predetermined cellular phenotype. In some implementations, at least a portion of the functional data 122 includes results from flow cytometry. Table 420 in Figure 4B provides example of functional data 122.

In some implementations, the respective functional data 122 includes (824) information obtained from live cells extracted from a tumor site (e.g., cancerous cells extracted from a diseased site) of the respective patient 110, and the respective functional data 122 includes one or more of a size distribution of the live cells, a shape distribution of the live cells, a distribution of the live cells with respect to expression of a biomarker, and phenotypic features of the live cells. In some implementations, the respective functional data 122 includes additional distributions with respect to expression of one or more biomarkers. In some implementations, the respective functional data 122 includes specific biomarkers that are associated with the first drug therapy. In some implementations, at least a portion of the functional data 122 includes results from flow cytometry. Table 420 in Figure 4B provides example of functional data 122.

In some implementations, the respective functional data 122 includes (826) information obtained from live cells extracted from a tumor site (e.g., cancerous cells extracted from a diseased site) of the respective patient 110, and the first drug therapy (e.g., drug therapy 150) includes at least a first drug, and the respective functional data 122 includes one or more of: a measure of the potency (e.g., IC₅₀) of one or more first drugs for inhibiting a predetermined biochemical function, a maximum cytotoxicity of the one or more first drugs, an area under a curve (AUC) determined based on data (e.g., raw data) corresponding to cell viability in response to dosage of the one or more first drugs (e.g., drug sensitivity), and the one or more first drugs includes at least the first drug. Table 440 of Figure 4D provides an example of functional data 122, and graph 430 of Figure 4C illustrates raw data corresponding to cell viability in response to dosage of the one or more first drugs plotted as a line graph.

In some implementations, such as when the first drug therapy 150-1 includes a predefined combination of two or more drugs, the one or more drugs includes drugs of the predetermined combination of two or more drugs. For example, when the first drug therapy 150-1 includes a predefined combination of two or more drugs, the one or more drugs includes all drugs that are included in the first drug therapy. In another example, when the first drug therapy 150-1 includes a predefined combination of two or more drugs, the one or more drugs includes at least one drug that is included in the first drug therapy. In yet another example, when the first drug therapy 150-1 includes a predefined combination of two or more drugs, the one or more drugs includes at least one drug that is included in the first drug therapy and the one or more drugs may also include additional drugs that are not included in the first drug therapy.

In some implementations, for each patient of a second plurality of patients (870), the device retrieves (872) respective functional data 122 and respective clinical data 124 corresponding to the respective patient of the second plurality of patients. The respective functional data 122 corresponding to the respective patient of the second plurality of patients includes (874) initial cell viability 412 and cell viability in response to exposure to one or more drug therapies (e.g., drug sensitivity). The respective functional data 122 corresponding to the respective patient of the second plurality of patients data includes (876) one or more of: a measure of the potency (e.g., IC₅₀) of one or more second drugs for inhibiting a predetermined biochemical function, a maximum cytotoxicity of the one or more second drugs, and an area under a curve (AUC) determined using a plot (e.g., graph 430) of cell viability in response to dosage of the one or more second drugs. The one or more second drugs differs (878) from the one or more first drugs by at least one drug, the one or more second drugs includes a second drug that is different from the first drug, and the respective clinical data 124 corresponding to the respective patient of the second plurality of patients includes patient information over time. The device forms (880) a respective feature vector that includes the respective functional data 122 and respective clinical data 124 corresponding to the respective patient of the second plurality of patients. The device uses (882) at least a second subset of the feature vectors corresponding to the respective patient of the second plurality of patients to train a second model (e.g., predictive model 132-2) to predict individual patient response 152-2 to a second drug therapy that is different from the first drug therapy (e.g., second drug therapy 150-2 that is different from the first drug therapy 150-1). The device then stores (884) the trained second model (e.g., predictive model 132-2) in a database (e.g., database 240, 290) for subsequent use in predicting patient response to the second drug therapy. The second drug therapy is distinct from the first drug therapy and includes at least the second drug.

In some implementations, the computer stores (886) the trained first model (e.g., predictive model 132-1) and the trained second model (e.g., predictive model 132-2) in a database for subsequent use in predicting patient response to a third drug therapy (e.g., drug therapy 150-3) that includes at least the first drug of the first drug therapy (e.g., drug therapy 150-1) and the second drug of the second drug therapy (e.g., drug therapy 150-2). For example, the first drug therapy 150-1 may include the first drug and may include any number of drugs (e.g., one drug, 2 drugs, 3 drugs, etc.). The second drug therapy 150-2 may include the second drug and may include any number of drugs (e.g., one drug, 2 drugs, 3 drugs, etc.). The third drug therapy 150-3 includes the first drug, the second drug, and optionally, any drugs in addition to the first and second drugs.

In some implementations, the respective clinical data 124 includes (828) one or more of: an age of the respective patient 110, a sex of the respective patient 110, a weight of the respective patient 110, a diagnosis date, patient information over time, an indicator regarding whether or not the patient 110 has relapsed, an indicator of the respective patient's response 514 to a second drug therapy, a stage of the respective patient's disease progression, a concentration of total protein 516, a concentration of one or more biochemicals, an indicator of the drug therapy (e.g., chemotherapy 512) the respective patient 110 is receiving, a tumor size, and an indication of other health conditions associated with the respective patient. In some implementations, the second drug therapy may be the same as the first drug therapy (e.g., is the same chemotherapy, includes the same one or more drugs). In some implementations, the second drug therapy is different from the first drug therapy (e.g., differs from the first drug therapy by at least one drug). For instance, the second drug therapy includes at least one drug that is not included in the first drug therapy. The second drug therapy may include one or more drugs that overlap with one or more drugs in the first drug therapy. In some implementations, the clinical data 124 also includes a concentration of one or more biomarkers that are known to be associated with the first drug therapy.

In some implementations, the one or more drug therapies 150 are (804) one or more chemotherapies, and each chemotherapy includes one or more drugs for treating cancer.

In some implementations, the device determines (862) that each of the respective functional data 122 and respective clinical data 124 is complete, and in accordance with a determination that at least one of the respective functional data 122 and respective clinical data 124 includes (864) one or more missing values, the device replaces at least one of the one or more missing values with an inferred value. For example, when the functional data 122 is missing one or more values, at least one of the missing values is replaced with an inferred value that is determined using k-nearest neighbors algorithm where k is any integer of any value, such as 8, 9, 10, 11, 12, etc.

In some implementations, the feature vectors are used (851) to train the first model (e.g., predictive model 132-m) to output a prediction interval 154 corresponding to the predicted individual patient response 152 to the first drug therapy 150.

In some implementations, the first drug therapy (e.g., drug therapy 150-1) includes (852) a predefined combination of two or more drugs (e.g., a predetermined cocktail or two or more anti-cancer drugs).

In some implementations, the first subset of the feature vectors (e.g., training data 310) is (852) a subset, less than all, of the feature vectors. The device uses a second subset of the feature vectors (e.g., testing data 312), distinct from the first subset of the feature vectors, to test the trained model (e.g., predictive model in-training 320).

In some implementations, at least a first subset of the plurality of patients includes patients that have undergone (853) one or more drug therapies that includes the first drug therapy.

In some implementations, the one or more drug therapies associated with the first subset of the plurality of patients includes (854) one or more drug therapies that are different from the first drug therapy.

In some implementations, the plurality of patients further include a second subset of patients that have undergone (856) one or more drug therapies that includes drugs other than the first drug.

In some implementations, the plurality of patients further includes a second subset of patients that have undergone (857) one or more drug therapies that are different from the one or more drug therapies associated with the first subset of patients, and the one or more drug therapies associated with the second subset of patients do not include the first drug therapy.

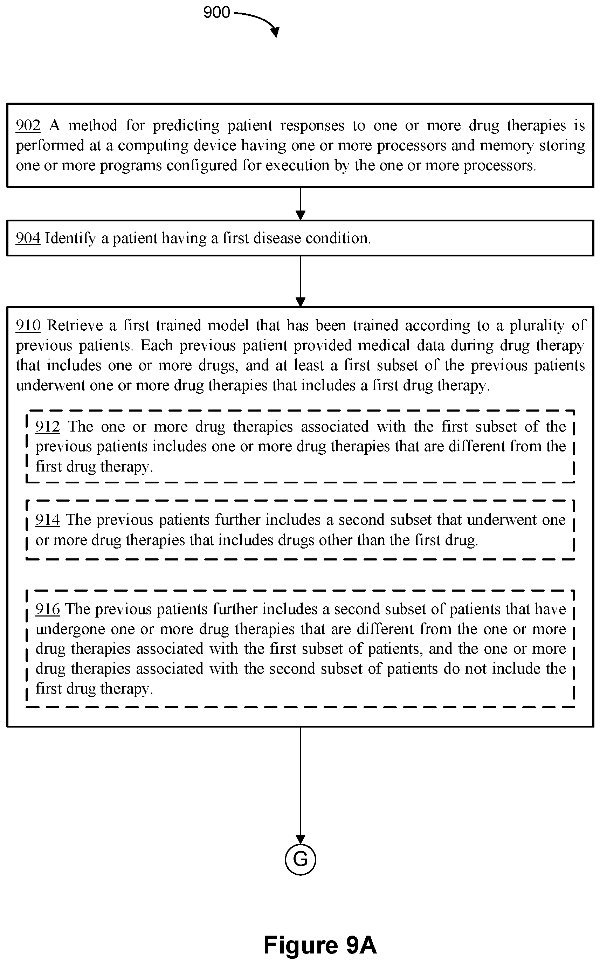

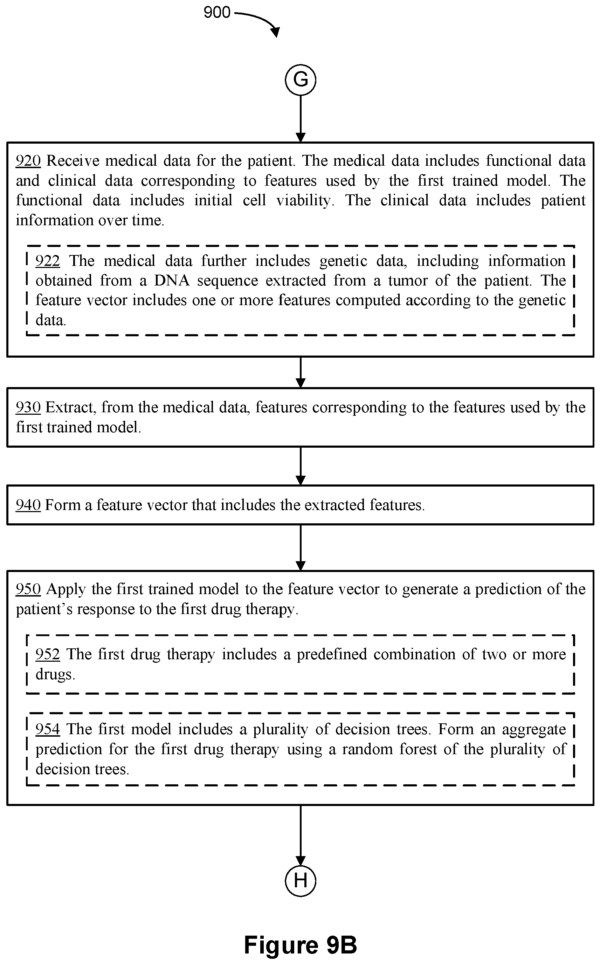

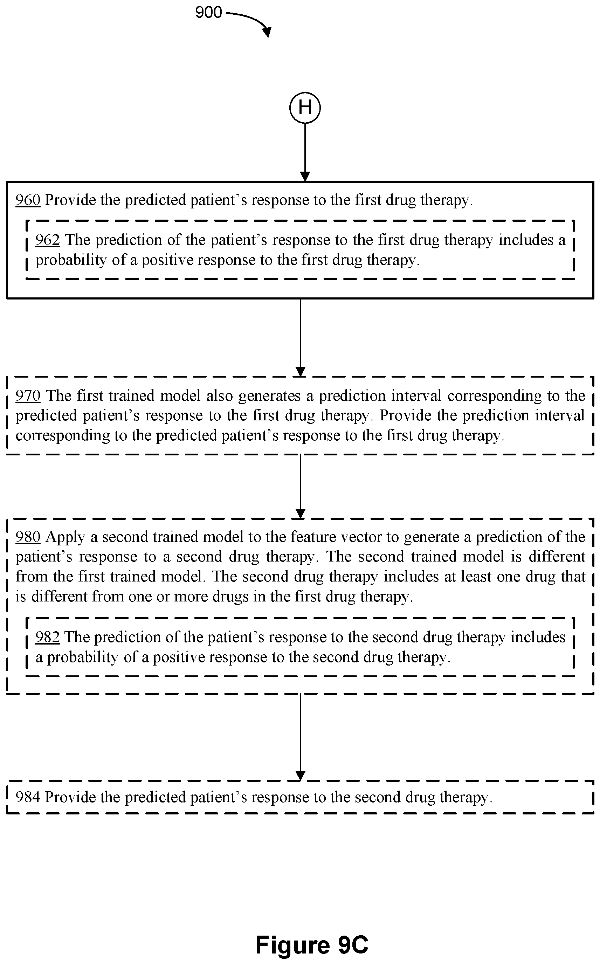

Figures 9A - 9C provide a flow diagram of a method 900 for matching patients to clinicians according to some implementations. The steps of the method 900 may be performed by a computer system, corresponding to a computer device 200 or a server 250. In some implementations, the computer includes one or more processors and memory. Figures 9A - 9C correspond to instructions stored in a computer memory or computer-readable storage medium (e.g., the memory 206 of the computing device 200). The memory stores one or more programs configured for execution by the one or more processors. For example, the operations of the method 900 are performed (902), at least in part, by one or more predictive models 132.

In accordance with some implementations, a computer system or computing device 200 identifies (904) a patient (e.g., new patient 140) having a first disease condition (e.g., cancer, a type of cancer), and retrieves (910) a first trained model (e.g., predictive model 132, such as predictive model 132-1) built to (e.g., trained to) predict patient response 152 to a first drug therapy 150 (such as first drug therapy 150-1) for treating the first disease condition. The first trained model 132 has been trained according to data for a plurality of previous patients 110. Each previous patient 110 provided medical data (e.g., patient data 120) during drug therapy (e.g., chemotherapy, treatments) that includes one or more drugs, and at least a first subset of the previous patients 110 underwent one or more drug therapies (e.g., one or more chemotherapies) that includes the first drug therapy. The computer then receives (920) medical data 141 for the patient 140. The medical data 141 includes functional data 142 and clinical data 144 corresponding to features used by the first trained model 132-1. The functional data 142 includes initial cell viability 412, and the clinical data 144 includes patient information over time (e.g., response 514). The computer extracts (930), from the medical data 141, features corresponding to the features used by the first trained model 132-1. The device then forms a feature vector comprising the extracted features, applies the first trained model 132-1 to the feature vector to generate a prediction 152 (e.g., 152-1) of the patient's response to the first drug therapy 150 (e.g., 150-1), and provides the predicted patient's response 152 to the first drug therapy 150. Figure 1B illustrates an example of receiving medical data 141 (e.g., new patient data 141) corresponding to the new patient 140 at the first trained model 132 and providing a predicted patient response 152-1 to the first drug therapy 150-1. Examples of predicted patient responses 152 to different drug therapies 150 is provided with respect to Figures 7A and 7B. Details and examples regarding functional data 142 and clinical data 144 are provided with respect to Figures 4A - 4D and Figure 5, respectively.

In some implementations, the functional data 142 also includes cell viability in response to exposure to one or more drug therapies (e.g., drug sensitivity information, as shown in Figures 4C and 4D).

In some implementations, the medical data 141 further includes (922) genetic data 146, including information obtained from a DNA sequence extracted from a tumor of the patient (e.g., extracted from cancerous cells), and the feature vector includes one or more features computed according to the genetic data 126.

In some implementations, the first trained model 132-1 also generates (970) a prediction interval 154-1 corresponding to the predicted patient's response 152-1 to the first drug therapy 150-1, and the computer provides the prediction interval 154-1 of the predicted patient's response 152-1 to the first drug therapy 150-1.

In some implementations, the prediction of the patient's response 152 to the first drug therapy 150 (e.g., drug therapy 150-1) includes (982) a probability (e.g., likelihood) of a positive response to the first drug therapy 150.

In some implementations, the computer applies (980) a second trained model 132-2 to the feature vector to generate a prediction of the patient's response 152-2 to a second drug therapy 150-2, and the computer provides (984) the predicted patient's response 152-2 to the second drug therapy 150-2. The second trained model 132-1 is different from the first trained model 132-1, and the second drug therapy 150-2 includes at least one drug that is different from one or more drugs in the first drug therapy 150-1. For example, the second trained model 132-2 may be trained using patient data corresponding to a second plurality of patients that is different from patient data 120 corresponding to a first plurality of patients used for training the first trained model 132-1. The second plurality of patients differs from the first plurality of patients by at least one patient. For example, the second plurality of patients may be a subset, less than all, of the first plurality of patients. Alternatively, the second plurality of patients may include at least one patient that is not included in the first plurality of patients. In some implementations, at least one of the patients of the first plurality of patients is the same as at least one patient of the second plurality of patients (e.g., some overlapping patients). In some implementations, patients of the second plurality of patients are not included in the first plurality of patients (e.g., non-overlapping patients).

In some implementations, the prediction of the patient's response 152-2 to the second drug therapy 150-2 includes (982) a probability (e.g., likelihood) of a positive response to the second drug therapy 150-2.

In some implementations, the first drug therapy 150 (e.g., drug therapy 150-1) includes (952) a predefined combination of two or more drugs. In such cases, the prediction of the patient's response 152 to the first drug therapy 150 includes a probability (e.g., likelihood) of a positive response to the combination of two or more drugs (e.g., patient response to receiving treatment or drug therapy that includes receiving the combination of two or more drugs).

In some implementations, the first model 132 (e.g., predictive model 132-1) includes (954) a plurality of decision trees, and the computer forms an aggregate prediction for the first drug therapy 150 (e.g., drug therapy 150-1) using a random forest of the plurality of decision trees.

In some implementations, the first model 132 (e.g., predictive model 132-1) is a support vector machine.

In some implementations, the first model 132-1 is a first type of machine learning model and the second model 132-2 is a second type of machine learning model that is different from the first type of machine learning model. For example, the first model 132-1 is a random forest that includes a plurality of decision trees and the second model 132-2 is a neural network that includes a plurality of layers. In another example, the first model 132-1 is a random forest that includes a plurality of decision trees and the second model 132-2 is a support vector machine.

In some implementations, the one or more drug therapies 150 associated with the first subset of the previous patients 110 includes (912) one or more drug therapies that are different from the first drug therapy 150-1. For example, a patient of the first subset of the previous patients 110 includes a patient who has not been (e.g., never been) treated with the first drug therapy 150-1. In some implementations, a patient of the first subset of the previous patients 110 includes a patient who been treated with at least one drug that is included in the first drug therapy 150-1 (e.g., the patient may have been treated with drug therapy 150-m that is different from the first drug therapy 150-1 and the drug therapy 150-m includes one or more drugs in common with the first drug therapy 150-1).

In some implementations, the previous patients 110 further include a second subset of patients that underwent (914) one or more drug therapies that includes drugs other than the first drug. For example, a patient of the first subset of the previous patients 110 includes a patient who has been treated with a drug that is not included in the first drug therapy 150-1.

In some implementations, the previous patients 110 further include a second subset that underwent (916) one or more drug therapies 150 that are different from the one or more drug therapies associated with the first subset, and the one or more drug therapies associated with the second subset do not include the first drug therapy. For example, a patient of the first subset of the previous patients 110 includes a patient who has not been (e.g., never been) treated with any drugs that are included in the first drug therapy 150-1.

The terminology used in the description herein is for the purpose of describing particular implementations only and is not intended to be limiting of the invention. As used in the description and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof.

The foregoing description, for purpose of explanation, has been described with reference to specific implementations. However, the illustrative discussions above are not intended to be exhaustive or to limit the invention to the precise forms disclosed. Many modifications and variations are possible in view of the above teachings. The implementations were chosen and described in order to best explain the principles of the invention and its practical applications, to thereby enable others skilled in the art to best utilize the invention and various implementations with various modifications as are suited to the particular use contemplated. The present invention is defined by the claims which follow.

## Claims

1. A method of predicting patient response to a first drug therapy, performed at a computing device having one or more processors and memory storing one or more programs configured for execution by the one or more processors:
identifying a patient having a first disease condition;
retrieving a first trained machine learning model built to predict response to the first drug therapy for treating the first disease condition, wherein:
the first trained machine learning model has been trained according to data for a plurality of previous patients;
each previous patient provided medical data during drug therapy that includes one or more drugs, wherein the first trained machine learning model was trained using a respective feature vector for each previous patient comprising respective functional data including initial cell viability and cell viability in response to exposure to one or more drug therapies, and respective clinical data including patient information over time; and
at least a first subset of the previous patients underwent one or more drug therapies that include the first drug therapy;
receiving medical data for the patient, the medical data including functional data (122) and clinical data (124) corresponding to features used by the first trained machine learning model, wherein:
the functional data includes initial cell viability (412) and cell viability in response to one or more drug therapies; and
the clinical data includes patient information over time comprising a plurality of recorded patient responses to drug therapy;
extracting, from the medical data, features corresponding to the features used by the first trained machine learning model;
forming a feature vector (830) comprising the extracted features:
applying the first trained machine learning model to the feature vector to generate a prediction of the patient's response to the first drug therapy; and
providing the prediction of the patient's response to the first drug therapy.

2. The method of claim 1, wherein:
the medical data further includes genetic data, including information obtained from a DNA sequence extracted from a tumor of the patient; and
the feature vector includes one or more features computed according to the genetic data.

3. The method of claim 1, wherein the first trained machine learning model also generates a prediction interval corresponding to the prediction of the patient's response to the first drug therapy, the method further comprising:
providing the prediction interval for the prediction of the patient's response to the first drug therapy.

4. The method of claim 1, wherein the prediction of the patient's response to the first drug therapy includes a probability of a positive response to the first drug therapy.

5. The method of claim 1, further comprising:
applying a second trained machine learning model to the feature vector to generate a prediction of the patient's response to a second drug therapy; and
providing the prediction of the patient's response to the second drug therapy, wherein
the second trained machine learning model is different from the first trained machine learning model; and
the second drug therapy includes at least one drug that is different from one or more drugs in the first drug therapy.

6. The method of claim 5, wherein the prediction of the patient's response to the second drug therapy includes a probability of a positive response to the second drug therapy.

7. The method of claim 1, wherein the first drug therapy includes a predefined combination of two or more drugs.

8. The method of claim 1, wherein the first machine learning model includes a plurality of decision trees, and the method further comprises forming an aggregate prediction for the first drug therapy using a random forest of the plurality of decision trees.

9. The method of claim 1, wherein the one or more drug therapies associated with the first subset of the previous patients includes one or more drug therapies that are different from the first drug therapy.

10. The method of claim 1, wherein:
the first drug therapy includes a first drug; and
the previous patients further include a second subset that underwent one or more drug therapies that includes drugs other than the first drug.

11. The method of claim 1, wherein the previous patients further include a second subset that underwent one or more drug therapies that are different from the one or wmore drug therapies associated with the first subset, and the one or more drug therapies associated with the second subset do not include the first drug therapy.

12. The method of claim 1, further comprising:
for each patient of the plurality of previous patients:
retrieving respective functional data and respective clinical data corresponding to the respective patient, wherein:
the respective functional data includes initial cell viability and cell viability in response to exposure to the one or more drug therapies; and
the respective clinical data includes patient information over time;
forming a respective feature vector comprising the respective functional data and the respective clinical data corresponding to the respective patient;
using at least a first subset of the feature vectors to train a first model to predict individual patient response to the first drug therapy; and
storing the trained first model in a database as the first trained machine learning model for subsequent use in predicting patient response to the first drug therapy.

13. A non-transitory computer readable storage medium storing one or more programs configured for execution by a computer system having one or more processors, memory, and a display, the one or more programs comprising instructions for performing any of the methods of claims 1 - 11.

14. A computer system for predicting patient response to one or more drug therapies, the computer system comprising:
one or more processors;
memory; and
one or more programs stored in the memory and configured for execution by the one or more processors, the one or more programs comprising instructions for performing any of the methods of claims 1 - 11.

## Patentansprüche

1. Verfahren zum Vorhersagen des Ansprechens eines Patienten auf eine erste Arzneimitteltherapie, das auf einer Recheneinrichtung durchgeführt wird, die einen oder mehrere Prozessoren und einen Speicher aufweist, in dem ein oder mehrere Programme gespeichert sind, die zur Ausführung durch den einen oder die mehreren Prozessoren eingerichtet sind:
Identifizieren eines Patienten mit einem ersten Krankheitszustand;
Abrufen eines ersten trainierten Modells für maschinelles Lernen, das zur Vorhersage des Ansprechens auf die erste Arzneimitteltherapie zur Behandlung des ersten Krankheitszustands erstellt wurde, wobei:
das erste trainierte Modell für maschinelles Lernen anhand von Daten für eine Vielzahl früherer Patienten trainiert wurde;
jeder frühere Patient während einer Arzneimitteltherapie, die ein oder mehrere Arzneimittel umfasst, medizinische Daten bereitstellte, wobei das erste trainierte Modell für maschinelles Lernen unter Verwendung eines jeweiligen Merkmalsvektors für jeden früheren Patienten trainiert wurde, wobei der jeweilige Merkmalsvektor jeweilige funktionelle Daten einschließlich einer anfänglichen Zellvitalität und einer Zellvitalität als Reaktion auf eine Exposition gegenüber einer oder mehreren Arzneimitteltherapien sowie jeweilige klinische Daten einschließlich Patienteninformationen im Zeitverlauf umfasst; und
mindestens eine erste Teilmenge der früheren Patienten einer oder mehreren Arzneimitteltherapien unterzogen wurde, die die erste Arzneimitteltherapie umfassen;
Empfangen medizinischer Daten für den Patienten, wobei die medizinischen Daten funktionelle Daten (122) und klinische Daten (124) umfassen, die Merkmalen entsprechen, die von dem ersten trainierten Modell für maschinelles Lernen verwendet werden, wobei:
die funktionellen Daten eine anfängliche Zellvitalität (412) und eine Zellvitalität als Reaktion auf eine oder mehrere Arzneimitteltherapien umfassen; und
die klinischen Daten Patienteninformationen im Zeitverlauf umfassen, die eine Vielzahl aufgezeichneter Reaktionen des Patienten auf eine Arzneimitteltherapie umfassen;
Extrahieren von Merkmalen aus den medizinischen Daten, wobei die Merkmale den von dem ersten trainierten Modell für maschinelles Lernen verwendeten Merkmalen entsprechen;
Bilden eines Merkmalsvektors (830), der die extrahierten Merkmale umfasst;
Anwenden des ersten trainierten Modells für maschinelles Lernen auf den Merkmalsvektor, um eine Vorhersage des Ansprechens des Patienten auf die erste Arzneimitteltherapie zu erzeugen; und
Bereitstellen der Vorhersage des Ansprechens des Patienten auf die erste Arzneimitteltherapie.

2. Verfahren nach Anspruch 1, wobei:
die medizinischen Daten ferner genetische Daten umfassen, einschließlich Informationen, die aus einer DNA-Sequenz gewonnen wurden, die aus einem Tumor des Patienten extrahiert wurde; und
der Merkmalsvektor ein oder mehrere anhand der genetischen Daten berechnete Merkmale umfasst.

3. Verfahren nach Anspruch 1, wobei das erste trainierte Modell für maschinelles Lernen ferner ein Vorhersageintervall erzeugt, das der Vorhersage des Ansprechens des Patienten auf die erste Arzneimitteltherapie entspricht, wobei das Verfahren ferner umfasst:
Bereitstellen des Vorhersageintervalls für die Vorhersage des Ansprechens des Patienten auf die erste Arzneimitteltherapie.

4. Verfahren nach Anspruch 1, wobei die Vorhersage des Ansprechens des Patienten auf die erste Arzneimitteltherapie eine Wahrscheinlichkeit eines positiven Ansprechens auf die erste Arzneimitteltherapie umfasst.

5. Verfahren nach Anspruch 1, ferner umfassend:
Anwenden eines zweiten trainierten Modells für maschinelles Lernen auf den Merkmalsvektor, um eine Vorhersage des Ansprechens des Patienten auf eine zweite Arzneimitteltherapie zu erzeugen; und
Bereitstellen der Vorhersage des Ansprechens des Patienten auf die zweite Arzneimitteltherapie, wobei
das zweite trainierte Modell für maschinelles Lernen von dem ersten trainierten Modell für maschinelles Lernen verschieden ist; und
die zweite Arzneimitteltherapie mindestens ein Arzneimittel umfasst, das sich von einem oder mehreren Arzneimitteln in der ersten Arzneimitteltherapie unterscheidet.

6. Verfahren nach Anspruch 5, wobei die Vorhersage des Ansprechens des Patienten auf die zweite Arzneimitteltherapie eine Wahrscheinlichkeit eines positiven Ansprechens auf die zweite Arzneimitteltherapie umfasst.

7. Verfahren nach Anspruch 1, wobei die erste Arzneimitteltherapie eine vordefinierte Kombination von zwei oder mehr Arzneimitteln umfasst.

8. Verfahren nach Anspruch 1, wobei das erste Modell für maschinelles Lernen eine Vielzahl von Entscheidungsbäumen umfasst und das Verfahren ferner das Bilden einer aggregierten Vorhersage für die erste Arzneimitteltherapie unter Verwendung eines Random Forest aus der Vielzahl von Entscheidungsbäumen umfasst.

9. Verfahren nach Anspruch 1, wobei die der ersten Teilmenge der früheren Patienten zugeordneten Arzneimitteltherapien eine oder mehrere Arzneimitteltherapien umfassen, die von der ersten Arzneimitteltherapie verschieden sind.

10. Verfahren nach Anspruch 1, wobei:
die erste Arzneimitteltherapie ein erstes Arzneimittel umfasst; und
die früheren Patienten ferner eine zweite Teilmenge umfassen, die einer oder mehreren Arzneimitteltherapien unterzogen wurde, welche andere Arzneimittel als das erste Arzneimittel umfassen.

11. Verfahren nach Anspruch 1, wobei die früheren Patienten ferner eine zweite Teilmenge umfassen, die einer oder mehreren Arzneimitteltherapien unterzogen wurde, welche sich von den Arzneimitteltherapien unterscheiden, die der ersten Teilmenge zugeordnet sind, und wobei die Arzneimitteltherapien, die der zweiten Teilmenge zugeordnet sind, die erste Arzneimitteltherapie nicht umfassen.

12. Verfahren nach Anspruch 1, ferner umfassend:
für jeden Patienten aus der Vielzahl früherer Patienten:
Abrufen jeweiliger funktioneller Daten und jeweiliger klinischer Daten, die dem jeweiligen Patienten entsprechen, wobei:
die jeweiligen funktionellen Daten eine anfängliche Zellvitalität und eine Zellvitalität als Reaktion auf eine Exposition gegenüber der einen oder den mehreren Arzneimitteltherapien umfassen; und
die jeweiligen klinischen Daten Patienteninformationen im Zeitverlauf umfassen;
Bilden eines jeweiligen Merkmalsvektors, der die jeweiligen funktionellen Daten und die jeweiligen klinischen Daten, die dem jeweiligen Patienten entsprechen, umfasst;
Verwenden mindestens einer ersten Teilmenge der Merkmalsvektoren zum Trainieren eines ersten Modells, um das individuelle Ansprechen eines Patienten auf die erste Arzneimitteltherapie vorherzusagen; und
Speichern des trainierten ersten Modells in einer Datenbank als das erste trainierte Modell für maschinelles Lernen zur späteren Verwendung bei der Vorhersage des Ansprechens eines Patienten auf die erste Arzneimitteltherapie.

13. Nichtflüchtiges computerlesbares Speichermedium, auf dem ein oder mehrere Programme gespeichert sind, die zur Ausführung durch ein Computersystem mit einem oder mehreren Prozessoren, einem Speicher und einer Anzeige eingerichtet sind, wobei das eine oder die mehreren Programme Anweisungen zum Ausführen eines der Verfahren nach den Ansprüchen 1 bis 11 umfassen.

14. Computersystem zum Vorhersagen des Ansprechens eines Patienten auf eine oder mehrere Arzneimitteltherapien, wobei das Computersystem umfasst:
einen oder mehrere Prozessoren;
einen Speicher; und
ein oder mehrere Programme, die in dem Speicher gespeichert und zur Ausführung durch den einen oder die mehreren Prozessoren eingerichtet sind, wobei das eine oder die mehreren Programme Anweisungen zum Ausführen eines der Verfahren nach den Ansprüchen 1 bis 11 umfassen.

## Revendications

1. Procédé de prédiction de la réponse d'un patient à un premier traitement médicamenteux, mis en œuvre sur un dispositif informatique comportant un ou plusieurs processeurs et une mémoire stockant un ou plusieurs programmes configurés pour être exécutés par le ou les processeurs :
identification d'un patient présentant un premier état pathologique ;
récupération d'un premier modèle d'apprentissage automatique entraîné, élaboré pour prédire la réponse au premier traitement médicamenteux destiné à traiter le premier état pathologique, dans lequel :
le premier modèle d'apprentissage automatique entraîné a été entraîné à partir de données relatives à une pluralité de patients antérieurs ;
chaque patient antérieur a fourni, au cours d'un traitement médicamenteux comprenant un ou plusieurs médicaments, des données médicales, le premier modèle d'apprentissage automatique entraîné ayant été entraîné en utilisant un vecteur de caractéristiques respectif pour chaque patient antérieur, comprenant des données fonctionnelles respectives incluant une viabilité cellulaire initiale et une viabilité cellulaire en réponse à une exposition à un ou plusieurs traitements médicamenteux, ainsi que des données cliniques respectives incluant des informations relatives au patient au cours du temps ; et
au moins un premier sous-ensemble des patients antérieurs a été soumis à un ou plusieurs traitements médicamenteux comprenant le premier traitement médicamenteux ;
réception de données médicales relatives au patient, les données médicales comprenant des données fonctionnelles (122) et des données cliniques (124) correspondant à des caractéristiques utilisées par le premier modèle d'apprentissage automatique entraîné, dans lesquelles :
les données fonctionnelles comprennent une viabilité cellulaire initiale (412) et une viabilité cellulaire en réponse à un ou plusieurs traitements médicamenteux ; et
les données cliniques comprennent des informations relatives au patient au cours du temps, lesquelles comprennent une pluralité de réponses enregistrées du patient à un traitement médicamenteux ;
extraction, à partir des données médicales, de caractéristiques correspondant aux caractéristiques utilisées par le premier modèle d'apprentissage automatique entraîné ;
formation d'un vecteur de caractéristiques (830) comprenant les caractéristiques extraites ;
application du premier modèle d'apprentissage automatique entraîné au vecteur de caractéristiques afin de générer une prédiction de la réponse du patient au premier traitement médicamenteux ; et
fourniture de la prédiction de la réponse du patient au premier traitement médicamenteux.

2. Procédé selon la revendication 1, dans lequel :
les données médicales comprennent en outre des données génétiques, incluant des informations obtenues à partir d'une séquence d'ADN extraite d'une tumeur du patient ; et
le vecteur de caractéristiques comprend une ou plusieurs caractéristiques calculées à partir des données génétiques.

3. Procédé selon la revendication 1, dans lequel le premier modèle d'apprentissage automatique entraîné génère également un intervalle de prédiction correspondant à la prédiction de la réponse du patient au premier traitement médicamenteux, le procédé comprenant en outre :
fourniture de l'intervalle de prédiction pour la prédiction de la réponse du patient au premier traitement médicamenteux.

4. Procédé selon la revendication 1, dans lequel la prédiction de la réponse du patient au premier traitement médicamenteux comprend une probabilité d'une réponse positive au premier traitement médicamenteux.

5. Procédé selon la revendication 1, comprenant en outre :
application d'un second modèle d'apprentissage automatique entraîné au vecteur de caractéristiques afin de générer une prédiction de la réponse du patient à un second traitement médicamenteux ; et
fourniture de la prédiction de la réponse du patient au second traitement médicamenteux, dans lequel
le second modèle d'apprentissage automatique entraîné est différent du premier modèle d'apprentissage automatique entraîné ; et
le second traitement médicamenteux comprend au moins un médicament différent d'un ou de plusieurs médicaments du premier traitement médicamenteux.

6. Procédé selon la revendication 5, dans lequel la prédiction de la réponse du patient au second traitement médicamenteux comprend une probabilité d'une réponse positive au second traitement médicamenteux.

7. Procédé selon la revendication 1, dans lequel le premier traitement médicamenteux comprend une combinaison prédéfinie de deux médicaments ou plus.

8. Procédé selon la revendication 1, dans lequel le premier modèle d'apprentissage automatique comprend une pluralité d'arbres de décision, et le procédé comprend en outre la formation d'une prédiction agrégée pour le premier traitement médicamenteux à l'aide d'une forêt aléatoire constituée de la pluralité d'arbres de décision.

9. Procédé selon la revendication 1, dans lequel le ou les traitements médicamenteux associés au premier sous-ensemble des patients antérieurs comprennent un ou plusieurs traitements médicamenteux différents du premier traitement médicamenteux.

10. Procédé selon la revendication 1, dans lequel :
le premier traitement médicamenteux comprend un premier médicament ; et
les patients antérieurs comprennent en outre un second sous-ensemble ayant été soumis à un ou plusieurs traitements médicamenteux comprenant des médicaments autres que le premier médicament.

11. Procédé selon la revendication 1, dans lequel les patients antérieurs comprennent en outre un second sous-ensemble ayant été soumis à un ou plusieurs traitements médicamenteux différents du ou des traitements médicamenteux associés au premier sous-ensemble, et le ou les traitements médicamenteux associés au second sous-ensemble ne comprennent pas le premier traitement médicamenteux.

12. Procédé selon la revendication 1, comprenant en outre :
pour chaque patient de la pluralité de patients antérieurs :
récupération de données fonctionnelles respectives et de données cliniques respectives correspondant au patient respectif, dans lesquelles :
les données fonctionnelles respectives comprennent une viabilité cellulaire initiale et une viabilité cellulaire en réponse à une exposition à un ou plusieurs des traitements médicamenteux ; et
les données cliniques respectives comprennent des informations relatives au patient au cours du temps ;
formation d'un vecteur de caractéristiques respectif comprenant les données fonctionnelles respectives et les données cliniques respectives correspondant au patient respectif ;
utilisation d'au moins un premier sous-ensemble des vecteurs de caractéristiques pour entraîner un premier modèle à prédire la réponse individuelle d'un patient au premier traitement médicamenteux ; et
stockage du premier modèle entraîné dans une base de données en tant que premier modèle d'apprentissage automatique entraîné, pour une utilisation ultérieure aux fins de prédiction de la réponse d'un patient au premier traitement médicamenteux.

13. Support de stockage non transitoire lisible par ordinateur sur lequel sont stockés un ou plusieurs programmes configurés pour être exécutés par un système informatique comportant un ou plusieurs processeurs, une mémoire et un dispositif d'affichage, le ou les programmes comprenant des instructions pour mettre en œuvre l'un quelconque des procédés selon les revendications 1 à 11.

14. Système informatique destiné à prédire la réponse d'un patient à un ou plusieurs traitements médicamenteux, le système informatique comprenant :
un ou plusieurs processeurs ;
une mémoire ; et
un ou plusieurs programmes stockés dans la mémoire et configurés pour être exécutés par le ou les processeurs, le ou les programmes comprenant des instructions pour mettre en œuvre l'un quelconque des procédés selon les revendications 1 à 11.
